# EUROPEAN PATENT SPECIFICATION

(11) **EP 0 770 876 B1**
(45) Date of publication and mention of the grant of the patent: **11.04.2001**
(21) Application number: 96610042.2
(22) Date of filing: 17.10.1996
(51) Int. Cl.: G01N 33/68, G01N 33/543, C12Q 1/37

(54) **A screening method for identifying ligands for target proteins**
Screening-Verfahren zur Ligand-Identifizierung von Zielproteinen
Méthode de screening pour l'identification de ligands des protéines cibles

(30) Priority: 25.10.1995 US 547889
(43) Date of publication of application: 02.05.1997
(73) Proprietor: Anadys Pharmaceuticals, Inc., Waltham, MA 02451 (US)
(72) Inventor: Pakula, Andrew, Lexington, Massachusetts 02173 (US); Bowie, James, Culver City, California 90232 (US)
(74) Representative: Christiansen, Ejvind

(56) References cited:
- EP-A- 0 613 007
- WO-A-92/03542
- WO-A-93/14781
- WO-A-97/20925
- WO-A-97/42500
- US-A- 4 859 609
- US-A- 5 506 097
- US-A- 5 585 277
- DATABASE WPI Section Ch, Week 7526 Derwent Publications Ltd., London, GB; Class B04, AN 75-43214W XP002024012 & JP-A-49 093 093 (AJINOMOTO KK) , 4 September 1974

## Description

This invention pertains to novel methods for high-throughput screening for pharmaceutical compounds, in particular those that bind to proteins involved in pathogenesis of disease or in regulation of a physiological function.

### Background of the Invention

Pharmaceuticals can be developed from lead compounds that are identified through a random screening process directed towards a target, such as a receptor. Large scale screening approaches can be complicated by a number of factors. First, many assays are laborious or expensive to perform. Assays may involve experimental animals, cell lines, or tissue cultures that are difficult or expensive to acquire or maintain. They may require the use of radioactive materials, and thus pose safety and disposal problems. These considerations often place practical limitations on the number of compounds that reasonably can be screened. Thus, those employing random screening methods are frequently forced to limit their search to those compounds for which some prior knowledge suggests that the compounds are likely to be effective. This strategy limits the range of compounds tested, and many useful drugs may be overlooked.

Furthermore, the specificity of many biochemical assays may exclude a wide variety of useful chemical compounds, because the interactions between the ligand and the receptor protein are outside the scope of the assay. For example, many proteins have multiple functions, whereas most assays are capable of monitoring only one such activity. With such a specific assay, many potential pharmaceuticals may not be detected.

Finally, in most existing biochemical screening approaches to drug discovery, the activity of the target protein must be defined. This requires that the system in question be well-characterized before screening can begin. Even when a protein sequence is known, as in e.g. a newly cloned gene, the specific functions of the protein may not be revealed simply by analysis of its sequence. Consequently, biochemical screening for therapeutic drugs directed against many target proteins must await detailed biochemical characterization, a process that generally requires extensive research.

Thus, there is a need in the art for a rapid, cost-effective, high-throughput assay that enables the screening of large numbers of compounds for their ability to bind therapeutically or physiologically relevant proteins. Furthermore, there is a need in the art for screening methods that are independent of the biological activity of the target proteins, and that will detect compounds that bind regions of the target proteins other than biologically active domains.

EP 865502 (prior art in accordance with Article 54(3)(4) EPC) discloses a method of identifying a ligand that binds to a predetermined target protein involving the contacting of test and control combinations with a conformation-sensitive fluorescence probe.

### Summary of the Invention

The present invention provides a method for identifying a ligand that binds a target protein. The method is carried out by:
(a) selecting as test ligands a plurality of compounds not known to bind to the target protein;
(b) incubating each of the test ligands and the target protein under conditions appropriate for the target protein to unfold to a appropriate extent, thereby producing a test combination;
(c) incubating the target protein as in step (b), but in the absence of a test ligand, to produce a control combination;
(d) determining the extent to which the target protein occurs in a folded state, an unfolded state, or both, in the test combination and in the control combination;
(e) comparing the determination made in step (d) between the test and control combinations, wherein if the target protein is present in the folded state to a greater or lesser extent in the test combination than in the control combination, the test ligand is a ligand that binds to the target protein; and
(f) repeating steps (b)-(e) with a plurality of said test ligands until at least one ligand that binds to the target protein is identified;
with the proviso that the incubation steps (b) and (c) do not comprise a contact of said test and control combinations with a conformational-sensitive fluorescence probe.

In practicing the present invention, any method may be used to determine the amount of target protein in folded or unfolded states, including without limitation proteolysis, antibody binding, surface binding, molecular chaperone binding, differential binding to immobilized ligand and differential formation of aggregated protein.

In one embodiment, the target protein is human Hemoglobin S (HbS), and ligands are identified by their ability to reduce the susceptibility of HbS to proteolysis.

### Brief Description of the Drawings

Figure 1 shows an SDS-polyacrylamide gel profile of carbonic anhydrase after proteolysis in the absence and presence of increasing concentrations of acetazolamide.

Figure 2 shows an SDS-polyacrylamide gel profile of carbonic anhydrase after proteolysis in the absence and presence of 1.0mM acetazolamide, in the absence and presence of a fungal extract.

Figure 3 shows a graph representing a titration of the binding of radiolabelled human neutrophil elastase to nitrocellulose filters after proteolysis in the absence and presence of increasing concentrations of elastatinal.

Figure 4 shows a graph representing a titration of the ELISA detection of human neutrophil elastase after proteolysis in the presence of increasing concentrations of ICI 200,355.

Figure 5 shows a graph representing the distribution of data for test ligands tested for binding to human neutrophil elastase.

Figure 6 shows a graph representing the titration of a ligand for human neutrophil elastase.

Figure 7 shows a graph representing the titration of five ligands for their ability to inhibit the enzymatic activity of human neutrophil elastase.

Figure 8 shows a graph representing a titration of the ELISA detection of human hemoglobin after proteolysis in the presence of increasing concentrations of 2,3-diphosphoglycerate.

Figure 9 shows a graph representing a titration of the binding of human hemoglobin to nitrocellulose filters after proteolysis in the absence or presence of increasing concentrations of 2,3-diphosphoglycerate.

Figure 10 shows a graph representing the distribution of binding data for test ligands tested for binding to human hemoglobin S.

Figure 11 shows a graph representing the titration of a ligand for human hemoglobin.

Figure 12 shows the structures of compounds identified as ligands for human hemoglobin S (HbS) and their activities in inhibiting HbS gelation relative to tryptophan (Trp).

Figure 13 shows a graph representing the ligand-binding activity for human hemoglobin of Zinc-bacitracin (BacZ), zinc-free bactracin (Bac), zinc-free bacitracin to which an equimolar concentration of ZnCl₂ has been added (Bac+Z), ZnCl₂, and zinc-bacitracin to which a molar excess of EDTA has been added (BacZ + EDTA).

### Detailed Description of the Invention

### Definitions

As used herein, the term "ligand" refers to an agent that binds a target protein. The agent may bind the target protein when the target protein is in its native conformation, or when it is partially or totally unfolded or denatured. According to the present invention, a ligand is not limited to an agent that binds a recognized functional region of the target protein e.g. the active site of an enzyme, the antigen-combining site of an antibody, the hormone-binding site of a receptor, a cofactor-binding site, and the like. In practicing the present invention, a ligand can also be an agent that binds any surface or internal sequences or conformational domains of the target protein. Therefore, the ligands of the present invention encompass agents that in and of themselves may have no apparent biological function, beyond their ability to bind to the target protein in the manner described above.

As used herein, the term "test ligand" refers to an agent, comprising a compound, molecule or complex, which is being tested for its ability to bind to a target protein. Test ligands can be virtually any agent, including without limitation metals, peptides, proteins, lipids, polysaccharides, nucleic acids, small organic molecules, and combinations thereof. Complex mixtures of substances such as natural product extracts, which may include more than one test ligand, can also be tested, and the component that binds the target protein can be purified from the mixture in a subsequent step.

As used herein, the term "target protein" refers to a peptide, protein or protein complex for which identification of a ligand or binding partner is desired. Target proteins include without limitation peptides or proteins known or believed to be involved in the etiology of a given disease, condition or pathophysiological state, or in the regulation of physiological function. Target proteins may be derived from any living organism, such as a vertebrate, particularly a mammal and even more particularly a human. For use in the present invention, it is not necessary that the protein's biochemical function be specifically identified. Target proteins include without limitation receptors, enzymes, oncogene products, tumor suppressor gene products, viral proteins, and transcription factors, either in purified form or as part of a complex mixture of proteins and other compounds. Furthermore, target proteins may comprise wild type proteins, or, alternatively, mutant or variant proteins, including those with altered stability, activity, or other variant properties, or hybrid proteins to which foreign amino acid sequences e.g. sequences that facilitate purification have been added.

As used herein, "test combination" refers to the combination of one or more test ligands and a target protein. "Control combination" refers to the target protein in the absence of a test ligand.

As used herein, the "folded state" of a protein refers to the native or undenatured form of the protein as it is present in its natural environment, or after isolation or purification, i.e. before exposure to denaturing conditions. This includes native proteins that may be detectably unfolded to differing extents in their natural environment, and whose folding patterns may change during their natural functioning. The "unfolded state" refers to a situation in which the polypeptide has lost elements of its secondary and/or tertiary structure that are present in its "folded state." It will be recognized by those skilled in the art that it is difficult to determine experimentally when a polypeptide has become completely unfolded i.e. has lost all elements of secondary and tertiary structure. Thus, the term "unfolded state" as used herein encompasses partial or total unfolding.

As used herein, "detectable fraction" refers to a quantity that is empirically determined and that will vary depending upon the method used to distinguish folded from unfolded protein. For example, when protease sensitivity is used to monitor folding, conditions are chosen (e.g. by adjusting temperature or adding denaturants) so that approximately 80% of the target protein is digested within a convenient incubation period. Alternatively, when antibodies specific to the folded or unfolded state of a target protein are used as the detection method, conditions are chosen so that a sufficient amount of antibody is bound to give a detectable signal.

The present invention encompasses high-throughput screening methods for identifying a ligand that binds a target protein. If the target protein to which the test ligand binds is associated with or causative of a disease or condition, the ligand may be useful for diagnosing, preventing or treating the disease or condition. A ligand identified by the present method can also be one that is used in a purification or separation method, such as a method that results in purification or separation of the target protein from a mixture. The present invention also relates to ligands identified by the present method and their therapeutic uses (for diagnostic, preventive or treatment purposes) and uses in purification and separation methods.

According to the present invention, a ligand for a target protein is identified by its ability to influence the extent of folding or the rate of folding or unfolding of the target protein. Experimental conditions are chosen so that the target protein is subjected to unfolding, whether reversible or irreversible. If the test ligand binds to the target protein under these conditions, the relative amount of folded:unfolded target protein or the rate of folding or unfolding of the target protein in the presence of the test ligand will be different, i.e. higher or lower, than that observed in the absence of the test ligand. Thus, the present method encompasses incubating the target protein in the presence and absence of a test ligand, under conditions in which (in the absence of ligand) the target protein would partially or totally unfold. This is followed by analysis of the absolute or relative amounts of folded vs. unfolded target protein or of the rate of folding or unfolding of the target protein.

An important feature of the present invention is that it will detect any compound that binds to any sequence or domain of the target protein, not only to sequences or domains that are intimately involved in a biological activity or function. The binding sequence, region, or domain may be present on the surface of the target protein when it is in its folded state, or may be buried in the interior of the protein. Some binding sites may only become accessible to ligand binding when the protein is partially or totally unfolded.

In practicing the present invention, the test ligand is combined with a target protein, and the mixture is maintained under appropriate conditions and for a sufficient time to allow binding of the test ligand to the target protein. Experimental conditions are determined empirically for each target protein. When testing test ligands, incubation conditions are chosen so that most ligand:target protein interactions would be expected to proceed to completion. In general, the test ligand is present in molar excess relative to the target protein. The target protein can be in a soluble form, or, alternatively, can be bound to a solid phase matrix. The matrix may comprise without limitation beads, membrane filters, plastic surfaces, or other suitable solid supports.

For each target protein, appropriate experimental conditions, e.g. temperature, time, pH, salt concentration, and additional components, are chosen so that a detectible fraction of the protein is present in an unfolded form in the absence of test ligand. For a target protein that unfolds irreversibly, preferred experimental conditions allow a detectable amount of the protein to unfold during a convenient incubation period in the absence of test ligand. To adjust or optimize the ratio of folded:unfolded protein or the rate of folding or unfolding, denaturing conditions may be required, including the use of elevated temperatures, the addition of chaotropes or denaturants such as urea or guanidium salts such as guanidinium thiocyanate, detergents, or combinations thereof. Furthermore, introduction of stabilizing or destabilizing amino acid substitutions may be used to manipulate the folded:unfolded ratio of target proteins.

The time necessary for binding of target protein to ligand will vary depending on the test ligand, target protein and other conditions used. In some cases, binding will occur instantaneously (e.g., essentially simultaneous with combination of test ligand and target protein), while in others, the test ligand-target protein combination is maintained for a longer time e.g. up to 12-16 hours, before binding is detected. When many test ligands are employed, an incubation time is chosen that is sufficient for most protein:ligand interactions.

Binding of a test ligand to the target protein is assessed by comparing the absolute amount of folded or unfolded target protein in the absence and presence of test ligand, or, alternatively, by determining the ratio of folded:unfolded target protein or the rate of target protein folding or unfolding in the absence and presence of test ligand. If a test ligand binds the target protein (i.e., if the test ligand is a ligand for the target protein), there may be significantly more folded, and less unfolded, target protein (and, thus, a higher ratio of folded to unfolded target protein) than is present in the absence of a test ligand. Alternatively, binding of the test ligand may result in significantly less folded, and more unfolded, target protein than is present in the absence of a test ligand. Similarly, binding of the test ligand may cause the rate of target protein folding or unfolding to change significantly.

In either case, determination of the absolute amounts of folded and unfolded target protein, the folded:unfolded ratio, or the rates of folding or unfolding, may be carried out using one of the known methods as described below. These methods include without limitation proteolysis of the target protein, binding of the target protein to appropriate surfaces, binding of specific antibodies to the target protein, binding of the target protein to molecular chaperones, binding of the target protein to immobilized ligands, and measurement of aggregation of the target protein. Other physico-chemical techniques may also be used, either alone or in conjunction with the above methods; these include without limitation measurements of circular dichroism, ultraviolet and fluorescence spectroscopy, and calorimetry. A preferred embodiment involves measuring the relative proteolysis of a target protein following incubation in the absence and presence of a test ligand. However, it will be recognized by those skilled in the art that each target protein may have unique properties that make a particular detection method most suitable for the purposes of the present invention.

For the purposes of high-throughput screening, the experimental conditions described above are adjusted to achieve a threshold proportion of test ligands identified as "positive" compounds or ligands from among the total compounds screened. This threshold is set according to two criteria. First, the number of positive compounds should be manageable in practical terms. Second, the number of positive compounds should reflect ligands with an appreciable affinity towards the target protein. A preferred threshold is achieved when 0.1% to 1% of the total test ligands are shown to be ligands of a given target protein.

Binding to a given protein is a prerequisite for pharmaceuticals intended to modify directly the action of that protein. Thus, if a test ligand is shown, through use of the present method, to bind a protein that reflects or affects the etiology of a condition, it may indicate the potential ability of the test ligand to alter protein function and to be an effective pharmaceutical or lead compound for the development of such a pharmaceutical. Alternatively, the ligand may serve as the basis for the construction of hybrid compounds containing an additional component that has the potential to alter the protein's function. In this case, binding of the ligand to the target protein serves to anchor or orient the additional component so as to effectuate its pharmaceutical effects. For example, a known compound that inhibits the activity of a family of related enzymes may be rendered specific to one member of the family by conjugation of the known compound to a ligand, identified by the methods of the present invention, that binds specifically to that member at a different site than that recognized by the known compound.

The fact that the present method is based on physico-chemical properties common to most proteins gives it widespread application. The present invention can be applied to large-scale systematic high-throughput procedures that allow a cost-effective screening of many thousands of test ligands. Once a ligand has been identified by the methods of the present invention, it can be further analyzed in more detail using known methods specific to the particular target protein used. For example, the ligand can be tested for binding to the target protein directly e.g. by incubating radiolabelled ligand with unlabelled target protein, and then separating protein-bound and unbound ligand. Furthermore, the ligand can be tested for its ability to influence, either positively or negatively, a known biological activity of the target protein.

In a preferred embodiment of the present invention, binding of test ligand to target protein is detected through the use of proteolysis. This assay is based on the increased susceptibility of unfolded, denatured polypeptides to protease digestion relative to that of folded proteins. In this case, the test ligand-target protein combination, and a control combination lacking the test ligand, are treated with one or more proteases that act preferentially upon unfolded target protein. After an appropriate period of incubation, the level of intact i.e. unproteolysed target protein is assessed using one of the methods described below e.g. gel electrophoresis and/or immunoassay.

There are two possible outcomes that indicate that the test ligand has bound the target protein. Either a significantly higher, or significantly lower, absolute amount of intact or degraded protein may be observed in the presence of ligand than in its absence.

Proteases useful in practicing the present invention include without limitation trypsin, chymotrypsin, V8 protease, elastase, carboxypeptidase, proteinase K, thermolysin, papain and subtilisin (all of which can be obtained from Sigma Chemical Co., St. Louis, MO). The most important criterion in selecting a protease or proteases for use in practicing the present invention is that the protease(s) must be capable of digesting the particular target protein under the chosen incubation conditions, and that this activity be preferentially directed towards the unfolded form of the protein. To avoid "false positive" results caused by test ligands that directly inhibit the protease, more than one protease, particularly proteases with different enzymatic mechanisms of action, can be used simultaneously or in parallel assays. In addition, cofactors that are required for the activity of the protease(s) are provided in excess, to avoid false positive results due to test ligands that may sequester these factors.

Typically, a purified target protein is first taken up to a final concentration of 1-100 µg/ml in a buffer containing 50 mM Tris-HCl, pH 7.5, 10% DMSO, 50 mM Nacl, 10% glycerol, and 1.0 mM DTT. Proteases, such as, for example, proteinase K or thermolysin (proteases with distinct mechanisms of action), are then added individually to a final concentration of 0.2-10.0 µg/ml. Parallel incubations are performed for different time periods ranging from 5 minutes to one hour, preferably 30 minutes, at 4°C, 15°C, 25°C, and 35°C. Reactions are terminated by addition of an appropriate protease inhibitor, such as, for example, phenylmethylsulfonyl chloride (PMSF) to a final concentration of 1mm (for serine proteases), ethylenediaminotetraacetic acid (EDTA) to a final concentration of 20 mM (for metalloproteases), or iodoacetamide (for cysteine proteases). The amount of intact protein remaining in the reaction mixture at the end of the incubation period may then be assessed by any method, including without limitation polyacrylamide gel electrophoresis, ELISA, or binding to nitrocellulose filters. It will be understood that additional experiments employing a narrower range of temperatures can be performed to establish appropriate conditions.

The above protocol allows the selection of appropriate conditions (e.g., protease concentration and digestion temperature) that result in digestion of approximately 70% of the target protein within a 30 minute incubation period, indicating that a significant degree of unfolding has occurred. Preferably, conditions are chosen so that proteolysis displays a temperature dependence indicative of a cooperative protein unfolding transition. To achieve this end, additional variables can be adjusted, including, for example, the concentrations of glycerol, salt, reducing agents, BSA or other "carrier proteins," target protein, denaturants and detergents. If a known ligand for the target protein is available, the ligand is included in the reaction mixture at a concentration above the Kd for its binding to the target protein and at least equal to the molar concentration of target protein, and the digestion experiment is repeated. Typically, at least a two-fold increase or decrease in the extent of digestion of the target protein is observed, indicating that binding of a known ligand changes the ratio of folded:unfolded target protein and/or the rate of folding or unfolding.

Once conditions are established for high-throughput screening as described above, the protocol is repeated simultaneously with a large number of test ligands at concentrations ranging from 20 to 200 µM. Observation of at least a two-fold increase or decrease in the extent of digestion of the target protein signifies a "hit" compound, i.e., a ligand that binds the target protein. Preferred conditions are those in which between 0.1% and 1% of test ligands are identified as "hit" compounds using this procedure.

In another embodiment, the relative amount of folded and unfolded target protein in the presence and absence of test ligand is assessed by measuring the relative amount of target protein that binds to an appropriate surface. This method takes advantage of the increased propensity of unfolded proteins to adhere to surfaces, which is due to the increased surface area, and decrease in masking of hydrophobic residues, that results from unfolding. If a test ligand binds a target protein (i.e., is a ligand of the target protein), it may stabilize the folded form of the target protein and decrease its binding to a solid surface. Alternatively, a ligand may stabilize the unfolded form of the protein and increase its binding to a solid surface.

In this embodiment, the target protein, a test ligand and a surface that preferentially binds unfolded protein are combined and maintained under conditions appropriate for binding of the target protein to a ligand and binding of unfolded target protein to the surface. Alternatively, the target protein and test ligand can be pre-incubated in the absence of the surface to allow binding. Surfaces suitable for this purpose include without limitation microtiter plates constructed from a variety of treated or untreated plastics, plates treated for tissue culture or for high protein binding, nitrocellulose filters and PVDF filters.

Determination of the amount of surface-bound target protein or the amount of target protein remaining in solution can be carried out using standard methods known in the art e.g. determination of radioactivity or immunoassay. If significantly more or less target protein is surface bound in the presence of a test ligand than in the absence of the test ligand, the test ligand is a ligand of the target protein. Similarly, the ratio of surface-bound:soluble target protein will be significantly greater or smaller in the presence of a test ligand than in its absence, if a test ligand is a ligand for the target protein.

In another embodiment, the extent to which folded and unfolded target protein are present in the test combination is assessed through the use of antibodies specific for either the unfolded state or the folded state of the protein i.e. denatured-specific ("DS"), or native-specific ("NS") antibodies, respectively. (Breyer, 1989, *J. Biol. Chem.,* **264 (5)**:13348-13354).

Polyclonal and monoclonal DS and NS antibodies specific for particular target proteins can be prepared by methods that are well known in the art (E. Harlow & D. Lane, *Antibodies: A Laboratory Manual,* Cold Spring Harbor Laboratory, 1988; Zola, *Monoclonal Antibodies: A Manual of* *Techniques,* CRC Press, Inc., Boca Raton, Florida,1987). For DS antibodies, animals can be immunized with a peptide from a region of the protein that is buried in the interior of the protein when it is in the native state. If the three-dimensional structure of the protein is unknown, antibodies are prepared against several peptides. Alternatively, fully denatured (i.e., unfolded) target protein is used as an immunogen.

The resulting antibodies are screened for preferential binding to the denatured state. For monoclonal antibodies, culture supernatants derived from individual cloned hybridomas are screened, and positive clones are used directly as a source of individual DS antibodies. For polyclonal antibodies, an unfractionated antiserum may exhibit preferential binding to the denatured state. Alternatively, DS antibodies may be purified from a polyclonal antiserum by selective adsorption techniques well-known in the art.

For NS antibodies, intact non-denatured protein, or one or more peptides known to be on the surface of the native protein, may be used as an immunogen. The resulting antibodies are screened as above for preferential binding to the native protein and purified for use in the present invention.

DS or NS antibodies can be utilized to detect a ligand-induced change in the level of folded target protein, unfolded target protein, the folded:unfolded ratio, or the rate of folding or unfolding.

In one approach, a test combination containing the DS antibody, the target protein, and the test ligand is exposed to a solid support e.g. a microtiter plate coated with the denatured target protein or a peptide fragment thereof, under conditions appropriate for binding of the target protein with its ligand and binding of the DS antibody to unfolded target protein. A control combination, which is the same as the test combination except that it does not contain test ligand, is processed in the same manner as the test solution. By comparing the amount of antibody bound to the plate or the amount remaining in solution in the test and control combinations, the difference in target protein folding is detected. The amount of antibody bound to the plate or remaining in solution can be measured as described below.

In a second approach, a test combination containing the DS antibody, the test ligand, and the target protein is exposed to a solid support coated with a second antibody, referred to as a solid phase antibody, which cannot bind to the target protein simultaneously with the DS antibody, and is specific for the target protein, but is either specific for the folded state (NS antibody) or unable to differentiate between the native and denatured states ("non-differentiating" or "ND" antibody). The resulting test combination or solution is maintained under conditions appropriate for binding of the target protein with a ligand of the target protein and for binding of the antibodies to the proteins they recognize. A control combination, which is the same as the test solution except that it does not contain test ligand, is processed in the same manner as the test solution. In both combinations, denatured (unfolded) target protein binds the DS antibody and is inhibited from binding the solid phase antibody. The ability of the test ligand to bind the target protein can be gauged by determining the amount of target protein that binds to the solid phase antibody in the test solution and comparing it with the extent to which target protein binds to the solid phase antibody in the absence of test ligand, which in turn reflects the amount of target protein in the folded state. The amount of target protein bound to the plate via the second antibody or remaining in solution can be detected by the methods described below. This approach may be used in a comparable manner with NS antibody as the soluble antibody and DS or ND antibody on the solid phase.

In a third approach, a test solution containing the target protein and the test ligand is exposed to a solid support e.g. a microtiter plate that has been coated with a DS or NS antibody and maintained under conditions appropriate for binding of target protein to its ligand and for binding of the antibody to target protein. Alternatively, the antibody can be present on the surfaces of beads. The ability of the test ligand to bind the target protein is gauged by determining the extent to which target protein remains in solution (unbound to the antibody) or on the solid surface (bound to the antibody), or the ratio of the two, in the presence and in the absence of test ligand. Alternatively, the antibody can be present in solution and the target protein can be attached to a solid phase, such as a plate surface or bead surface.

In another embodiment, molecular chaperones are used to assess the relative levels of folded and unfolded protein in a test combination. Chaperones encompass known proteins that bind unfolded proteins as part of their normal physiological function. They are generally involved in assembling oligomeric proteins, in ensuring that certain proteins fold correctly, in facilitating protein localization, and in preventing the formation of proteinaceous aggregates during physiological stress (Hardy, 1991, *Science,* **251**:439-443). These proteins have the ability to interact with many unfolded or partially denatured proteins without specific recognition of defined sequence motifs.

One molecular chaperone, found in *E*. *coli,* is a protein known as SecB. SecB has a demonstrated involvement in export of a subset of otherwise unrelated proteins. Competition experiments have shown that SecB binds tightly to all the unfolded proteins tested, including proteins outside of its particular export subset, but does not appear to interact with the folded protein. Other chaperones suitable for use in the present invention include without limitation heat shock protein 70s, heat shock protein 90s, GroEI and GroES (Gething et al., *Nature 355:33*, 1992).

In this embodiment, a test combination containing the test ligand and the target is exposed to a solid support e.g. microtiter plate or other suitable surface coated with a molecular chaperone, under conditions appropriate for binding of target protein with its ligand and binding of the molecular chaperone to unfolded target protein. The unfolded target protein in the solution will have a greater tendency to bind to the molecular chaperone-covered surface relative to the ligand-stabilized folded target protein. Thus, the ability of the test ligand to bind target protein can be determined by determining the amount of target protein remaining unbound, or the amount bound to the chaperone-coated surface.

Alternatively, a competition assay for binding to molecular chaperones can be utilized. A test combination containing purified target protein, the test ligand, and a molecular chaperone can be exposed to a solid support e.g. a microtiter well coated with denatured (unfolded) target protein, under conditions appropriate for binding target protein with its ligand and binding of the molecular chaperone to unfolded target protein. A control combination, which is the same as the test combination except that it does not contain test ligand, is processed in the same manner. Denatured target protein in solution will bind to the chaperone and thus inhibit its binding to the denatured target protein bound to the support. Binding of a test ligand to the target protein will result in a difference in the amount of unfolded target protein, and, thus, more or less chaperone will be available to bind to the solid-phase denatured target protein than is the case in the absence of binding of test ligand. Thus, binding of test ligand can be determined by assessing chaperone bound to the surface or in solution in the test combination and in the control combination and comparing the results. In this assay, the chaperones are generally not provided in excess, so that competition for their binding can be measured.

Alternatively, a test combination containing the target protein, the test ligand and a molecular chaperone can be exposed to a solid support e.g. a microtiter well that has been coated with antisera or a monoclonal antibody specific for the folded target protein (NS antibody) and unable to bind the target protein bound to the chaperone. Unfolded target protein will bind chaperone in solution and thus be inhibited from binding the solid phase antibody. By detecting target protein in the solution or bound to the well walls and comparing the extent of either or both in an appropriate control (the same combination without the test ligand), the ability of the test ligand to bind target protein can be determined. If the test ligand is a ligand for the target protein, more or less target protein will be bound to the antisera or monoclonal antibody bound to the container surface in the test combination than in the control combination, and correspondingly more or less target protein will be present unbound (in solution) in the test combination than in the control combination.

In another embodiment, a known ligand, cofactor, substrate, or analogue thereof of the target protein is used to assay for the presence of folded target protein. The higher the fraction of protein in the folded form, the greater the amount of protein that is available to bind to a ligand that binds exclusively to the folded state. Consequently, if a protein has a known ligand, it is possible to increase or decrease the binding of the protein to the known ligand by adding a test ligand that binds another site on the protein. For example, binding of dihydrofolate reductase to methotrexate, a folic acid analogue, can be used to assess the level of folding of this enzyme.

In this approach, the ligand, cofactor, substrate, or analogue thereof known to bind to the target protein is immobilized on a solid substrate. A solution containing the target protein and test ligand is then added. An increase or decrease in the amount of target protein that binds to the immobilized compound relative to an identical assay in the absence of test ligand indicates that the test ligand binds the target protein. The amount of target protein bound to the solid substrate can be assessed by sampling the solid substrate or by sampling the solution.

In another embodiment, the amount of unfolded target protein in a test combination is assessed by measuring protein aggregation. For proteins that unfold irreversibly, unfolded protein often forms insoluble aggregates. The extent of protein aggregation can be measured by techniques known in the art, including without limitation light scattering, centrifugation, and filtration.

In this approach, target protein and test ligand are incubated and the amount of protein aggregation is measured over time or after a fixed incubation time. The extent of protein aggregation in the test mixture is compared to the same measurement for a control assay in the absence of test ligand. If a test ligand binds a target protein, the rate of unfolding of target protein will be lower or higher than in the absence of test ligand. For measurements over time, the rate of appearance of aggregated protein will be lower or higher if the test ligand is a ligand for the target protein than if it is not. For measurements at a fixed time, there will be more or less unfolded protein and correspondingly more or less aggregated protein if the test ligand is a ligand for the target protein than if it is not. Thus, the ability of a test ligand to bind a target protein can be determined by assessing the extent of protein aggregation in the presence and absence of test ligand.

It will be understood that the methods of the present invention can be applied to fragments of target proteins that constitute stable structural domains. As used herein, "domain" refers to a fragment of a target protein that retains a significant degree of native folded structure after isolation. In some cases, a native protein will be cleaved by a protease into one or more such domains when proteolytic digestion of the native protein is performed at, for example, a lower temperature than that at which complete digestion of the protein occurs. In this case, it may be advantageous to assay the binding of test ligands to each of the domains independently. This may be achieved by either or both of the following approaches. First, one or more individual domains of a target protein can be prepared for use as targets in the assays described above, either by subjecting the intact protein to controlled proteolysis followed by purification of domain-comprising fragments, or by directing the synthesis of such fragments, either *in vitro* or *in vivo,* from recombinant DNA molecules encoding domain-comprising fragments of the protein. Second, domain-specific detection may be used to quantify folding in a reaction mixture in which the intact protein serves as the target. Methods for domain-specific detection include without limitation the use of domain-specific antibodies and chemical or enzymatic methods which selectively label particular domains. Domain-specific antibodies may be prepared by any method known in the art. For example, polyclonal domain-specific antibodies may be raised by using as immunogens either the purified or recombinant domains described above or domain-specific synthetic peptides. Alternatively, a panel of monoclonal antibodies may be prepared against the intact protein, and tested for reaction with purified or recombinant domains.

The embodiments described above are summarized in Table 1.

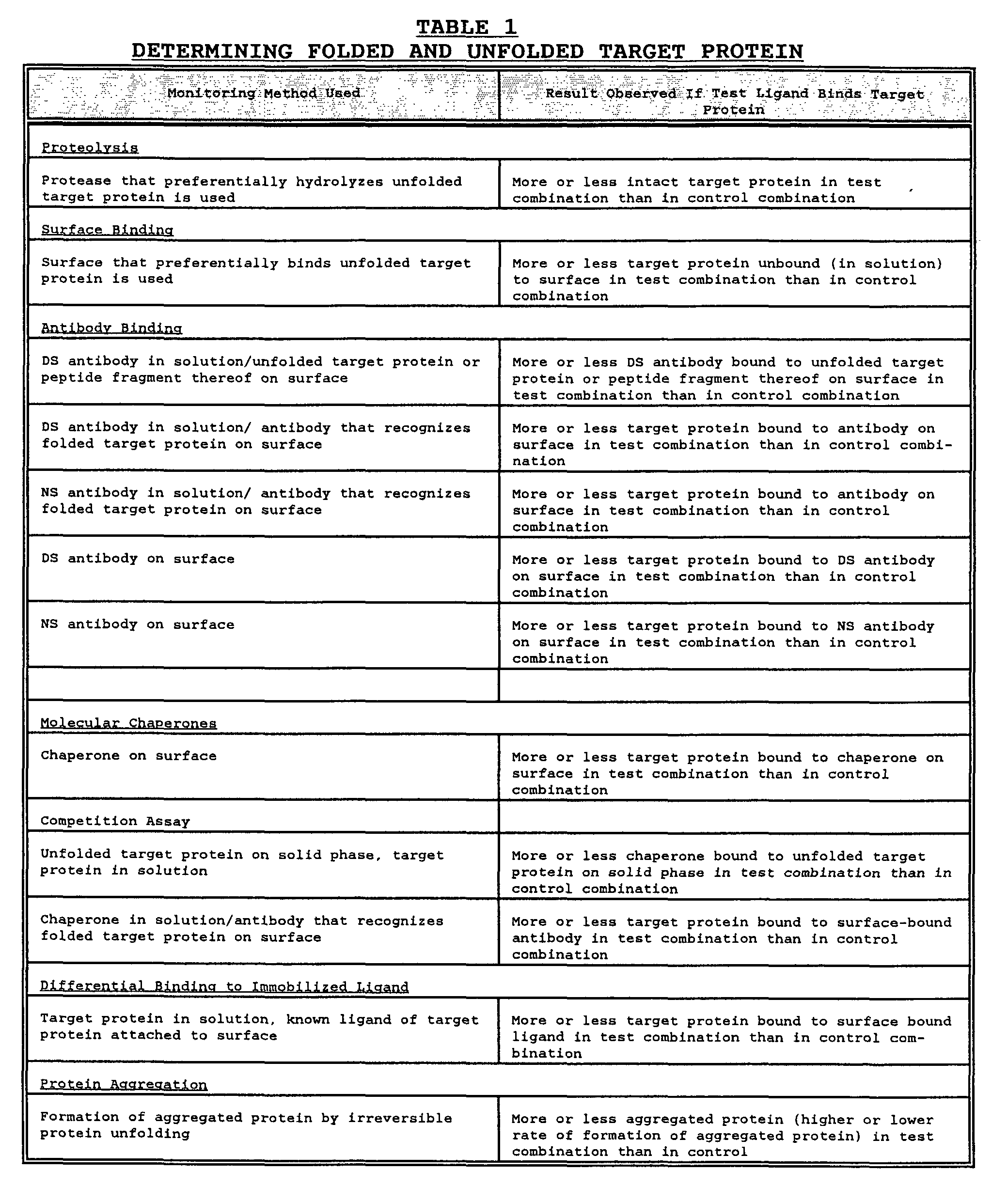

### Protein Detection Methods

The embodiments described above require a final step for detecting and/or quantifying the level of target protein or digestion products thereof, or antibodies, in order to quantify the relative amounts of folded and unfolded target protein after exposure to test ligands. In practicing the present invention, methods known in the art are used to detect the presence or absence of protein, small peptides or free amino acids. The method used will be determined by the product (proteins, peptides, free amino acids) to be detected. For example, techniques for detecting protein size can be used to determine the extent of proteolytic degradation of the target protein e.g. gel electrophoresis, capillary electrophoresis, size exclusion chromatography, high-performance liquid chromatography, and the like. Measurement of radioactivity, fluorescence, dye binding (Ciesiolka et al., *Anal.Biochem.* **168**:280, 1988), colloidal gold binding (Bradford, *Anal.Biochem.* **72**:248, 1976), or enzymatic activity can detect the presence or absence of products, either in solution or on a solid support. Immunological methods including e.g. ELISA and radioimmunoassay can detect the presence or absence of a known target protein in solution or on a substrate. The above methods are described in e.g. Harlow, E. and D. Lane, *Antibodies: A Laboratory Manual,* Cold Spring Harbor Laboratories, 1988; S.F.Y. Li, *Capillary Electrophoresis,* Elsevier Press, 1993; Bidlingmeyer, *Practical HPLC Methodology and Applications,* John Wiley and Sons, Inc., 1992; and Cantor, C.R. and P.R. Schimmel, *Biophysical Chemistry,* WH Freeman and Co., 1980.

In one embodiment, gel electrophoresis is used to detect the presence or absence of protein, and can further be used to detect the size of the protein. This latter method is especially useful in conjunction with proteolysis, as the presence of a greater or lesser amount of undigested target protein in the test combination than in the control combination indicates that the test ligand bound to the target protein.

The following examples are intended to illustrate the invention without limiting it thereof.

### Example 1: Methotrexate Binding Protects Dihydrofolate Reductase (DHFR) From Proteolytic Digestion by Proteinase K

The following were combined and incubated at 54°C for 5 minutes: DHFR (100 µg/ml), Proteinase K (80 µg/ml), 0.1 M Tris-HCl pH 7.5, and Methotrexate at 10⁻¹⁰ to 10⁻⁴ M.

Samples were removed and undigested DHFR was quantified by ELISA as follows:
(a) Protease incubations were diluted 50-fold with Tris-buffered saline (TBS);
(b) 50 µl diluted samples were transferred to the wells of an ELISA plate and incubated 60 minutes at room temperature;
(c) the plate wells were thoroughly washed with TBS plus 0.1% Tween-20 (TBST);
(d) 50 µl anti-DHFR rabbit serum diluted 250-fold into TBST plus 5% nonfat dry milk was added to each well and incubated 30 minutes at room temperature;
(e) plate wells were washed as in (c) above;
(f) 50 µl of goat anti-rabbit IgG alkaline phosphatase conjugate diluted 500-fold in TBST plus 5% milk was added to each well and incubated 30 minutes at room temperature;
(g) plate wells were washed as in (c); and
(h) 0.1 ml of 1.0 mg/ml *p*-nitrophenylphosphate in 0.1% diethanolamine was added. Color development is proportional to alkaline phosphatase antibody conjugate bound.

The ELISA analysis showed that methotrexate protects DHFR from digestion at concentrations of 10⁻⁸M and higher. By the same methods, nicotinamide adenine dinucleotide phosphate (NADPH) and dihydrofolate at concentrations of 10⁻⁵M and higher were shown to inhibit proteolysis of DHFR in separate experiments.

### Example 2: Methotrexate, NADPH and Dihydrofolate Binding Protects Dihydrofolate Reductase (DHFR) From Proteolytic Digestion by Proteinase K in the Presence of a Mixture of Amino Acids

The following were combined and incubated at 54' C for 5 minutes: DHFR (2.1 µg/ml), Proteinase K (80 µg/ml), 0.1M Tris-HCl (pH 7.5), 10⁻⁵M of all 20 common amino acids and either 0 or 10⁻⁵M ligand. The ligands used were the inhibitor Methotrexate and the substrates dihydrofolate and NADPH.

Samples were removed and undigested DHFR was quantified by ELISA as follows:
(a) Protease incubations were diluted 50 fold with Tris-buffered saline (TBS);
(b) 50 µl diluted samples were transferred to the wells of an ELISA plate and incubated 60 minutes at room temperature;
(c) the plate wells were thoroughly washed with TBS plus 0.1% Tween-20 (TBST);
(d) 50 µl anti-DHFR rabbit serum diluted 250 fold into TBST plus 5% nonfat dry milk was added to each well and incubated 30 minutes at room temperature;
(e) plate wells were washed as in (c) above;
(f) 50 µl of goat anti-rabbit IgG alkaline phosphatase conjugate diluted 500 fold in TBST plus 5% milk was added to each well and incubated 30 minutes at room temperature;
(g) plate wells were washed as in (c); and
(h) 0.1 ml of 1.0 mg/ml *p*-nitrophenylphosphate in 0.1% diethanolamine was added. Color development is proportional to alkaline phosphatase antibody conjugate bound.

The ELISA analysis showed that methotrexate and the substrates protect DHFR from digestion relative to the absence of ligands that bind to DHFR. Thus, specific binding can be detected in the presence of a complex mixture of compounds that do not bind to the target protein.

### Example 3: Methotrexate Binding Inhibits Binding of DHFR to Microtiter Plates

The following were combined in a volume of 60 µl and incubated in a Falcon 3072 "tissue-culture treated" microtiter plate at 20 or 47°C: 100 mg DHFR, 50 MM Tris-Cl (pH 7.5), and Methotrexate 10⁻¹⁰ to 10⁻⁴M.

50 µl of each sample was then transferred to the wells of an ELISA plate, and the DHFR that remained in solution was quantified by ELISA as follows:
(a) The 50 µl samples were incubated for 60 minutes at room temperature;
(b) the plate wells were thoroughly washed with TBS plus 0.1% Tween-20 (TBST);
(c) 50 µl anti-DHFR rabbit serum diluted 250-fold into TBST plus 5% nonfat dry milk was added to each well and incubated 30 minutes at room temperature;
(d) plate wells were washed as in (b) above;
(e) 50 µl of goat anti-rabbit IgG alkaline phosphatase conjugate diluted 500-fold in TBST plus 5% milk was added to each well and incubated 30 minutes at room temperature;
(f) plate wells were washed as in (b); and
(g) 0.1 ml of 1.0 mg/ml D-nitrophenylphosphate in 0.1% diethanolamine was added. Color development is proportional to alkaline phosphatase antibody conjugate bound.

The ELISA analysis revealed that methotrexate inhibits DHFR binding to the Falcon 3072 plate at concentrations of 10⁻⁷M and above.

### Example 4: Inhibition or Enhancement of Unfolded-Specific Antibody Binding

(1) ELISA plates are coated by incubation for 60 minutes with the following mixture: 4 µg/ml irreversibly denatured target protein or peptide fragments thereof in Tris-buffered Saline (10 mM Tris-Cl, pH 7.5, 0.2M NaCl; TBS).
(2) The plates are washed 3 times with TBS plus 0.1% Tween-20 (TBST).
(3) The following mixture (total volume 50 µl) is incubated in the coated wells of the microliter plate for 60 minutes:
   (a) Antibody specific for the unfolded state of the target protein at a sufficient concentration to give 50% of maximal binding (in the absence of competing target protein).
   (b) Target protein at a concentration sufficient to achieve 90% inhibition of antibody binding to the plate. The appropriate target protein concentration differs for each target protein. The concentration depends, in part, on the stability of the folded form of the target protein. In some cases it may be desirable to reduce the stability of the target protein by elevated temperature, inclusion of chemical protein-denaturing agents, or introduction of destabilizing amino acid substitutions in the target protein.
   (c) 10⁻⁹ to 10⁻⁵M test ligands
   (d) 5% nonfat dry milk in TBST
(4) The plates are washed 3 times with TBST.
(5) 50 µl of goat anti-IgG alkaline phosphatase conjugate at an appropriate dilution are added in TBST plus 5% nonfat dry milk and incubated for 30 minutes at room temperature.
(6) Plates are washed 3 times with TBST.
(7) 0.1 ml of 1.0 mg/ml D-nitrophenylphosphate in 0.1% diethanolamine are added and the amount of color development recorded by means of an ELISA plate reader.

ELISA analysis will reveal more or less antibody bound to the plate when successful test ligand-target protein binding has occurred than in the absence of such binding.

### Example 5: Inhibition or Enhancement of Chaperone Binding

(1) ELISA plates are coated by incubation for several hours with 4 µg/ml chaperone in TBS.
(2) The plates are washed 3 times with TBST.
(3) The following mixture (total volume 50 µl) is then incubated in the coated wells of the microtiter plate 10 for 60 minutes:
   (a) Target protein at a concentration sufficient to saturate about 50% of the available binding sites present on the chaperone proteins. Denaturing conditions may be used in cases where the folded form of the target protein is otherwise too stable to permit appreciable binding to chaperones.
   (b) 10⁻⁹ to 10⁻⁵M test ligands in TBST
(4) Aliquots of the well solutions are transferred to wells of a new ELISA plate and incubated for 60 minutes at room temperature.
(5) The plate wells are washed 3 times with TBST.
(6) 50 µl antibody specific for the target protein at the appropriate dilution in TBST, plus 5% nonfat dry milk, are added to each well and incubated 30 minutes at room temperature.
(7) The plate wells are washed 3 times with TBST.
(8) 50 µl of goat anti-rabbit IgG alkaline phosphatase conjugate at an appropriate dilution in TBST plus 5% nonfat dry milk are added to each well and incubated 30 minutes at room temperature.
(9) The plate wells are washed 3 times with TBST.
(10) 0.1 ml of 1.0 mg/ml *p*-nitrophenylphosphate in 0.1% diethanolamine will be added. Color development (proportional to alkaline phosphatase antibody conjugate bound) is monitored with an ELISA plate reader.

ELISA analysis will reveal target protein in the solution at higher or lower concentration when test ligand-target protein binding has occurred than when it has not.

### Example 6: Enhancement or Inhibition of Binding to a Known Ligand

(1) The following mixture (total volume 50 µl) is incubated in the coated wells of the microtiter plate for 60 minutes:
   (a) Ligand known to bind to the target protein, covalently attached to solid beads such as Sephadex. This ligand can be a small molecule or a macromolecule.
   (b) Target protein at a concentration well below saturation of the ligand and such that only 10% of the protein binds to the ligand sites. The solution conditions are such that most of the target protein is present in the denatured state.
   (c) 10⁻⁹ to 10⁻⁵M test ligands
   (d) in TBST plus necessary denaturant, such as urea.
(2) Aliquots of the well supernatant (free of beads) are transferred to wells of a new ELISA plate and incubated for 60 minutes at room temperature.
(3) The plate wells are washed 3 times with TBST.
(4) 50 µl antibody specific for the target protein at the appropriate dilution in TBST, plus 5% nonfat dry milk, are added to each well and incubated 30 minutes at room temperature.
(5) The plate wells are washed 3 times with TBST.
(6) 50 µl of goat anti-rabbit IgG alkaline phosphatase conjugate at an appropriate dilution in TBST plus 5% milk are added to each well and incubated 30 minutes at room temperature.
(7) The plate wells are washed 3 times with TBST.
(8) 0.1 ml of 1.0 mg/ml *p*-nitrophenylphosphate in 0.1% diethanolamine are added. Color development (proportional to alkaline phosphatase antibody conjugate bound) is monitored with an ELISA plate reader.

ELISA analysis will reveal a higher or lower concentration of target protein in the solution when successful test ligand-target protein binding has occurred.

### Example 7: Low Throughput Assay for HIV Rev Protein

Reaction mixtures (0.03 ml total volume) contained 30 µg/ml HIV Rev protein that had been produced in E. coli, 0.05M Tris-HCl, pH 7.5, 0.01M calcium acetate, 2.5µg/ml proteinase K, 10% DMSO, and varying amounts of tRNA as a known ligand. The reactions were incubated on ice for 15 minutes. After addition of PMSF and EDTA as described in Example 8 below, samples were prepared for gel electrophoresis and analyzed as described in Example 8.

The results showed that in the absence of tRNA, Rev protein is almost completely degraded by proteinase K under these conditions. In the presence of tRNA, however, a lower-molecular weight fragment of the protein is stabilized against proteolysis. Thus, binding of a known ligand to HIV Rev protein is detectable using the methods of the present invention.

### Example 8: Low throughput Assay for Carbonic Anhydrase Ligands

Ligand binding to carbonic anhydrase I (Sigma) was tested using proteolysis as a probe of target protein folding, and denaturing gel electrophoresis was used as a method for detection of intact protein remaining after digestion with proteases.

To validate the assay, acetazolamide, a known ligand of carbonic anhydrase, was tested. Though acetazolamide is a known inhibitor of carbonic anhydrase activity, these experiments make no use of that property, and do not measure the enzymatic activity of the protein. In addition, the sensitivity of the method to interference by a natural product extract was examined.

Reaction mixtures contained 13.3 µg/ml carbonic anhydrase, 0.05 M Tris-HCl pH 7.5, 0.01 M calcium acetate, 2.5 µg/ml proteinase K, 10% DMSO and acetazolamide (Sigma) in concentrations ranging from 0.0 to 1.0 mM. The reactions were incubated at 54°C for 15 minutes, and then chilled on ice. Phenyl methyl sulfonyl fluoride (PMSF) was then added from a 20 mM stock solution in ethanol to a final concentration of 1 mM, and EDTA was added from a 0.5M stock solution to a final concentration of 20 mM. 0.01 ml of SDS loading buffer (10% sodium dodecyl sulfate (SDS), 0.5 M Dithiothreitol, 0.4 M Tris-HCl buffer, pH 6.8, 50% Glycerol) was added and samples were heated at 95°C for 3 minutes. Samples were analyzed by SDS-polyacrylamide gel electrophoresis using a 4-15% polyacrylamide (BioRad) gradient gel, which was then stained with Coomassie Blue dye.

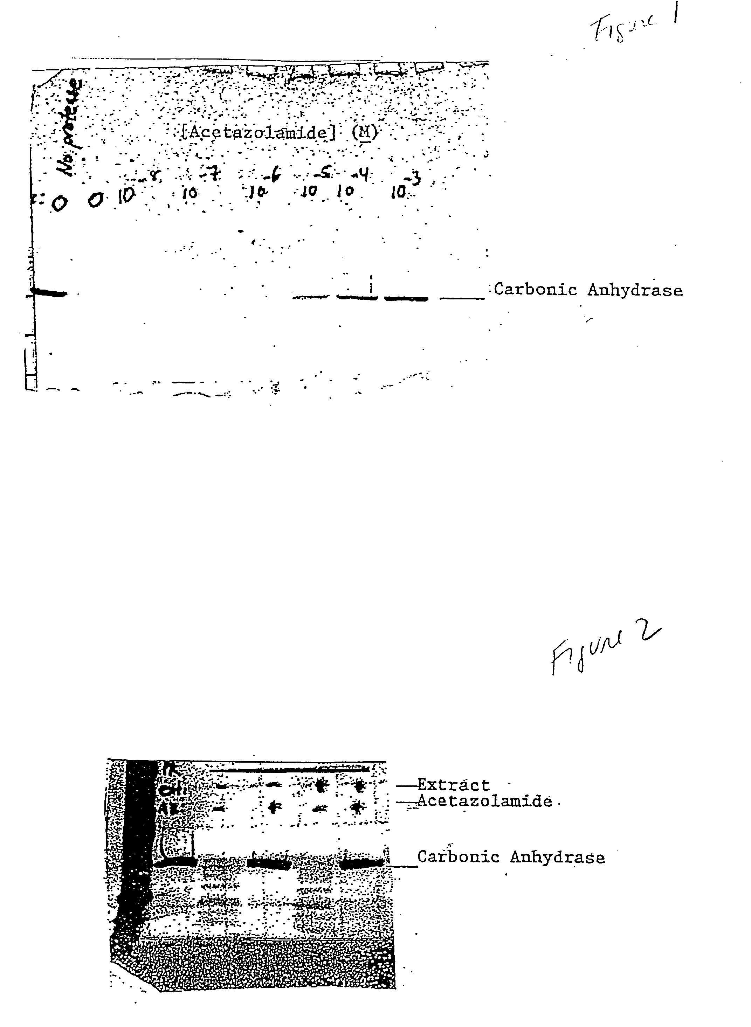

As shown in Figure 1, binding of the known ligand acetazolamide to carbonic anhydrase resulted in stabilization of carbonic anhydrase against proteolysis by proteinase K at 1 X 10⁻⁵M acetazolamide. The dissociation constant for this interaction has been reported to be 2.6 X 10⁻⁶M (Matsumoto, K. et. al. (1989), *Chem. Pharm. Bull,* **37**:1913-1915).

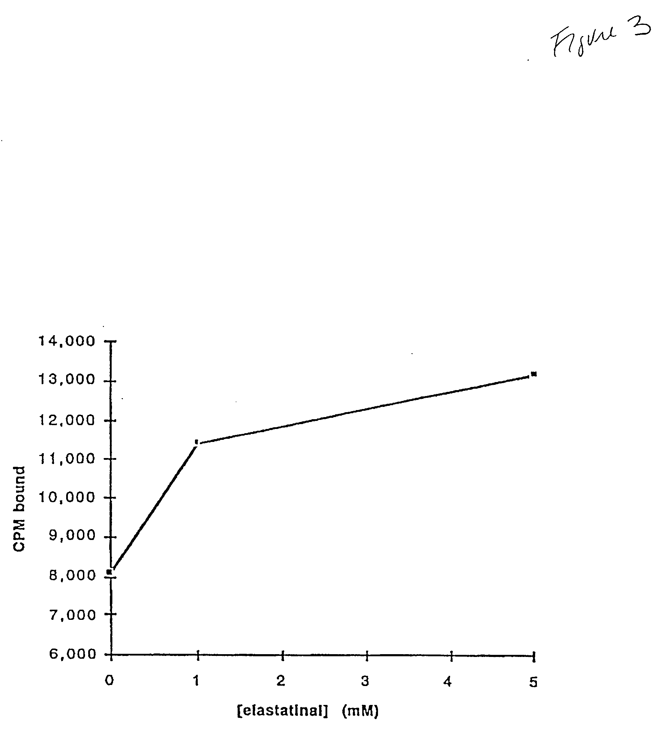

A fungal methanol extract was included in reactions that were otherwise identical to that described above such that the final concentration of an added small molecule would be equal to its concentration in the source culture. The presence of extract neither induced a false signal nor diminished the response to 1.0 mM acetazolamide (Figure 2.)

### Example 9: High-Throughput Screening of Ligands for Human Carbonic Anhydrase

A high throughput assay has been established for carbonic anhydrase I. Each reaction mixture (in a final volume of 0.05 ml) contains: 3.3 µg carbonic anhydrase, 50 mM Tris-HC1, 50 mM NaCl, 1.0 mM Ca(OAc)₂, and 0.13 µg proteinase K, 10% DMSO, and the appropriate test compound at a concentration of 20 µm. Control reactions are identical, except that the test compound is omitted. The mixtures are incubated at 20°C for 10 minutes, followed by incubation at 54°C for 30 minutes, after which they are placed on ice. Each mixture then receives 200 µl 50 mM sodium borate buffer, pH 8.5, containing 10 mM EDTA and 1.0 mM PMSF. After 20 minutes incubation on ice, 7.5 µl of the mixture are added to Dynatech Immulon-4 microplate wells containing 92.5 µl per well of the borate buffer described above. The plate is then incubated at 4°C overnight to permit carbonic anhydrase binding. Bound carbonic anhydrase is quantified by direct ELISA using HRP-conjugated anti-carbonic anhydrase I antibody at a 1:2,000 dilution (Biodesign, Kennebunk, ME); cat#K90046P) and Pierce (Rockford, IL) Turbo TMB substrate.

Carbonic anhydrase I inhibitors (obtained from Sigma Chemical Co., St. Louis, MO) were tested in the above assay over a range of inhibitor concentrations. Table 2 shows the concentration that caused 50% inhibition of proteolysis in the assay (IC₅₀), the published K_{d} values for the compounds (Matsumoto et al., *Chem. Pharm.* Bull., **37**: 1913, (1989)), and the ratio of these values for each compound. These data show a positive correlation between the IC₅₀ and K_{d} values for each inhibitor.

**TABLE 2**

| Ligand: | K_{d} (µM) | IC₅₀ (µM) | Ratio IC₅₀:K_{d} |
|---|---|---|---|
| Acetazolamide | 2.6 | 13 | 5.0 |
| Hydrochlorothiazide | 23.4 | 246 | 10.5 |
| Sulfanilamide | 36 | 350 | 9.7 |

### Example 10: High-throughput Screening of Ligands for Human Neutrophil Elastase

In practicing the present invention, the ability to perform the binding assay on large numbers of compounds is critical to its utility in discovering compounds with potential pharmaceutical utility. Two different approaches have been successfully implemented in a high-throughput screening mode and each of these has been applied to two target proteins: human neutrophil elastase (HNE) and human hemoglobin, both hemoglobin A (HbA) and hemoglobin S (HbS) (described in Example 11 below).

Notably, these target proteins differ from one another in a number of important respects: HbS is an intracellular, tetrameric protein that contains a prosthetic group critical to its function. It is known to exist in two conformations with different structural and functional properties. In contrast, HNE is monomeric, lacks a prosthetic group, and is secreted. HNE has an enzymatic activity (proteolysis) and does not appear to undergo any global conformational changes.

For high-throughput screening with both of these target proteins, proteolysis is used as the probe of target protein folding. The two high-throughput modes differ in the methods used for detection of residual target protein following proteolysis. The two detection methods are 1) capture of radiolabelled protein on nitrocellulose filters followed by quantitation of bound radioactivity and 2) measurement of protein by enzyme linked immunosorbent assay (ELISA.) Each of these methods was used successfully with both hemoglobin and HNE.

### A) Nitrocellulose binding of radiolabelled HNE:

0.1 mg HNE (Elastin Products) was labelled by reaction with ¹²⁵I-Sodium Iodide (Amersham) in the presence of Iodogen (Pierce) according to manufacturer's protocols (Pierce). Reaction mixtures were prepared in a final volume of 0.05 ml containing radiolabelled HNE (20,000 cpm, corresponding to approximately 10 µg), 0.025 mg/ml Bovine Serum Albumin, 50 mM Tris-HCl, pH 7.5, 10 mM calcium acetate, 2.5 µg/ml thermolysin (Boeringer Mannheim), 2.5 µg/ml proteinase K (Merck), 10% DMSO, and the test compound at a concentration of 200 µM. Control mixtures were identical, except that the test compound was omitted.

The mixtures were incubated at 20°C for 15 minutes, then at 65°C for 30 minutes, after which they were placed on ice. 0.12 ml 50 mM sodium acetate buffer, pH 4.5, was then added to each mixture. After an additional 15 minute incubation on ice, the samples were filtered through nitrocellulose membrane sheets using the Schleicher and Schuell Minifold. Each well of the apparatus was then washed once with 0.2 ml 50 mM sodium acetate buffer, pH 4.5, and twice with 0.5 ml 50 mM sodium phosphate, pH 5.5, containing 2.0% SDS and 1.0% Triton X-100. After drying the filter, bound radioactivity was determined by scintillation counting using the Wallac MicroBeta apparatus.

To validate the assay, a known ligand for HNE, elastatinal, was included in the assay at concentrations ranging from 1-5 mM. As shown in Figure 3, inclusion of elastatinal increased the retention of labelled HNE on the nitrocellulose filters, indicating that it protected HNE from proteolysis.

### B) ELISA Quantitation of HNE:

Reaction mixtures in final volume of 0.05 ml contained 2 µg/ml HNE, 0.020 mg/ml Bovine Serum Albumin, 50 mM Tris-HCl, pH 7.5, 10 mM calcium acetate, 7.5 µg/ml thermolysin (Boeringer Mannheim), 7.5 µg/ml proteinase K (Merck), 10% DMSO, and the test compound at 20 or 200 µM concentration. Control mixtures were identical except that the test compound was omitted. The mixtures were incubated at 20°C for 15 minutes, then at 63°C, 30 minutes then placed on ice.

0.1 ml of rabbit anti HNE antibody (Calbiochem) at a dilution of 1:10,000 in TBST (10 mM Tris-HCl, pH 7.5, 0.15 M NaCl, 0.05% Tween-20) containing 5% nonfat dry milk (Carnation) was then added to each reaction. After 10 minutes incubation at room temperature, the mixtures were transferred to 96-well Immulon-4 plates (Dynatech) that had been coated with HNE by overnight incubation with 0.1 ml per well of 0.2 µg/ml HNE in 50 mM Sodium Borate buffer, pH 8.5, and 3 mM sodium azide and then washed thoroughly with TBST. The plates were then incubated at room temperature for one hour, after which they were thoroughly washed with TBST. 0.1 ml of alkaline phosphatase-conjugated goat anti-rabbit IgG antibody (Calbiochem) diluted 1:1000 in TBST containing 5% nonfat dry milk was added to each well, and the plates were incubated at room temperature for 1-2 hours. The plates were then washed thoroughly with TBST and finally with TBST lacking Tween. 0.1 ml per ml of *p*-nitrophenylphosphate (0.5 mg/ml) in 1X diethanolamine substrate buffer (Pierce) was added to each well. Plates were incubated at room temperature until color developed, after which the absorbance of each well at 405 nm was measured using a BioRad 3550-UV microplate reader.

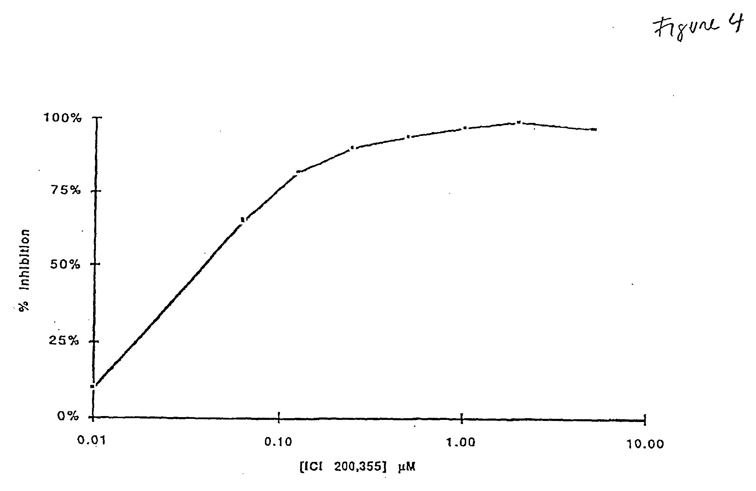

To validate the assay, a known ligand for HNE, ICI 200,355, was included in the assay at concentrations ranging from 0.01-10 µM. As shown in Figure 4, inclusion of the ligand caused an inhibition of antibody binding to the plate, indicating an increased level of immunoreactive HNE in the reaction mixtures.

### C) Results of High-Throughput Screening:

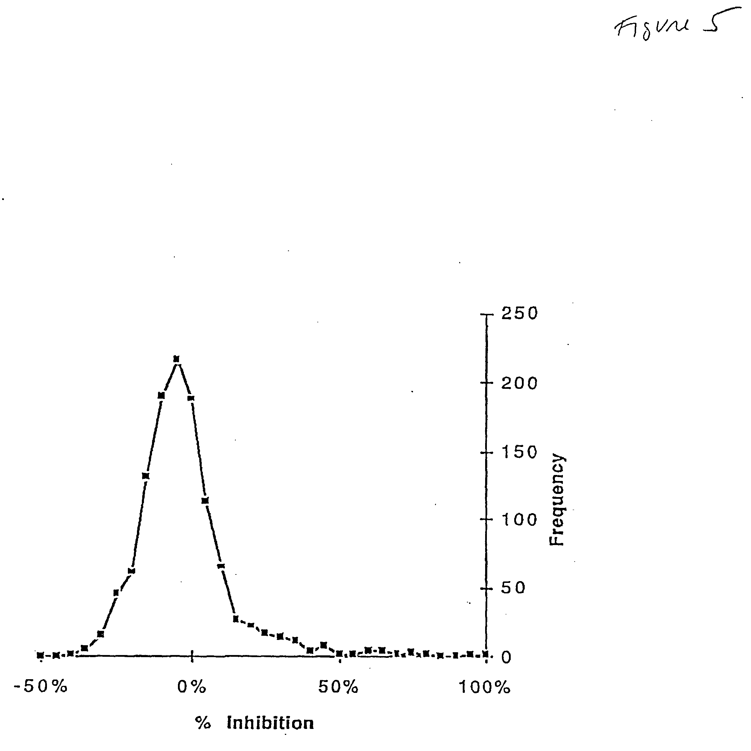

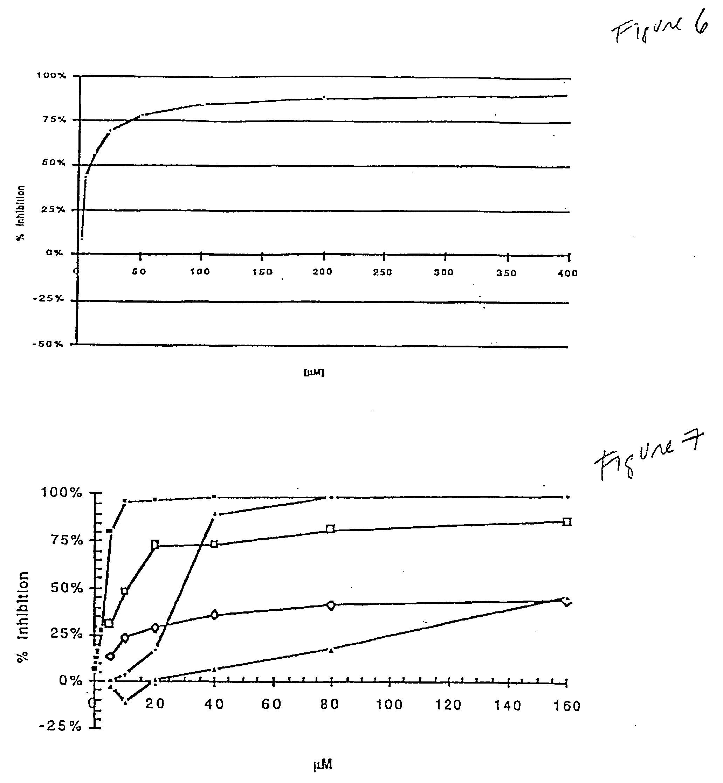

3,600 compounds have been screened for interaction with HNE using proteolysis and ELISA detection as above (Figure 5). Of these, 24 inhibited proteolysis of HNE by proteinase K to an extent of 50% or more when assayed at a concentration of 20 µM (positive hit compounds.) An additional 6 compounds were found to increase the extent of proteolysis at least two-fold when tested at 20 µM (negative hit compounds.) The concentration dependence of the effects of hit compounds was measured. Hit compounds showed half maximal effects at concentrations as low as 8 µM; one example is shown in Figure 6. Maximal inhibition was usually, but not always, nearly 100%.

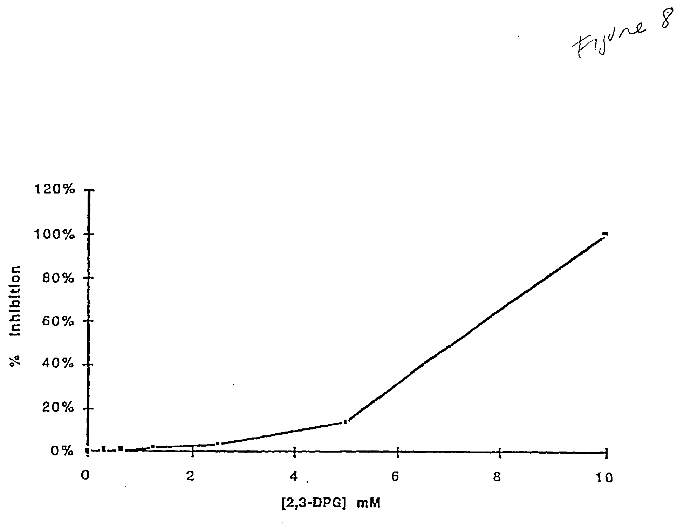

The hit compounds were assayed for their ability to inhibit the enzymatic activity of HNE. Since compounds identified in the binding assay may bind anywhere on the protein surface, only a small fraction would be expected to inhibit the enzymatic activity of HNE. The compounds were tested as inhibitors of the proteolysis of Suc-(Ala)₃-pNA (Elastin Products Co., Owensville, MI), a chromogenic synthetic substrate, according to the method of Bieth, J, Spiess, B. and Wermuth, C. G. (1974, *Biochemical Medicine,* **11**:350-357.) Two positive hit compounds and one negative hit compound inhibit the proteolytic activity of HNE significantly in these assays (Figure 7).

### D) Comparison of Binding Activity and Inhibitory Activity of Known HNE Inhibitors:

Three non-covalent inhibitors of human neutrophil elastase catalytic activity were obtained from Marion Merrell Dow, Inc. (Cincinnati, OH). Each of these compounds was tested for HNE binding activity over a range of concentrations using the methods of the present invention. Binding activity (IC₅₀) is expressed as the concentration required to inhibit proteolysis by 50%.

The compounds were also assayed for potency in inhibiting the catalytic activity of HNE. For this purpose, cleavage of the chromogenic substrate methoxysuccinyl-ala-ala-pro-val-p-nitroanilide (Elastin Products Co.) was monitored. In this assay, 100 µl reactions were prepared containing the chromogenic substrate (1 mM), 100 nM elastase, 1% DMSO, 100 mM Tris-HCl, pH 7.5, and 0.5 M NaCl. Cleavage was monitored over time by measuring the increase in absorbance of the reaction mixtures at 405 nm.

The IC₅₀ values reflecting binding to HNE, the IC₅₀ values for inhibition of HNE catalytic activity, and the ratio of these values are shown in Table 3.

**TABLE 3**

| Compound | Binding^{a} (µM) | Inhibition of Catalytic Activity^{b}(µM) | Ratio of Binding/Inhibition |
|---|---|---|---|
| MDL 101,146 | 1.2 | 0.22 | 5.5 |
| MDL 105,373 | 12 | 35 | 0.3 |
| MDL 103,900 | 1.5 | 1.4 | 1.1 |

| | | | |
|---|---|---|---|
| ^{a} expressed as IC₅₀ for inhibition of proteolysis of HNE | | | |
| ^{b} expressed as IC₅₀ for inhibition of HNE activity | | | |

These data reveal a close correlation between binding to HNE (as detected by the methods of the present invention) and inhibition of HNE catalytic activity for each of the inhibitors.

### Example 11: High-throughput Screening of Ligands for Human Hemoglobin

### A) Nitrocellulose Binding of Radiolabelled Hemoglobin:

0.2 mg HbS or HbA (Sigma) was radiolabelled by reaction with 1 mCi ¹²⁵I-Bolton-Hunter reagent (Amersham) in 100 mM sodium borate buffer, pH 8.5, on ice for one hour. Labelling was stopped by addition of borate buffer containing 200 mM glycine. The mixture was then fractionated by size on an execellulose GF-5 column (Pierce) in 50 mM sodium phosphate buffer, pH 7.5, containing 0.25% gelatin.

For the binding assay, reaction mixtures in a final volume of 0.05 ml contained radiolabelled hemoglobin (20,000 CPM), 0.063 mg/ml unlabelled hemoglobin, 0.034 mg/ml Bovine Serum Albumin, 50 mM Tris-HCl, pH 7.5, 10 mM calcium acetate, 2.5 µg/ml thermolysin (Boeringer Mannheim), 2.5 µg/ml proteinase K (Merck), 10% DMSO, and test compound. Control mixtures were identical, except that the test compound was omitted. The mixtures were incubated at 20°C for 15 minutes, then 40°C for 30 minutes and then placed on ice. 0.12 ml 50 mM sodium acetate buffer, pH 4.5, was then added to each mixture. After an additional 15 minute incubation on ice, the samples were filtered through nitrocellulose membrane sheets using the Schleicher and Schuell Minifold. Each well of the apparatus was then washed once with 0.2 ml 50 mM sodium acetate buffer, pH 4.5, twice with 0.5 ml of 50 mM sodium phosphate buffer, pH 5.5, containing 2.0% SDS and 1.0% Triton X-100. After drying the filter, bound radioactivity was determined by scintillation counting using the Wallac MicroBeta apparatus.

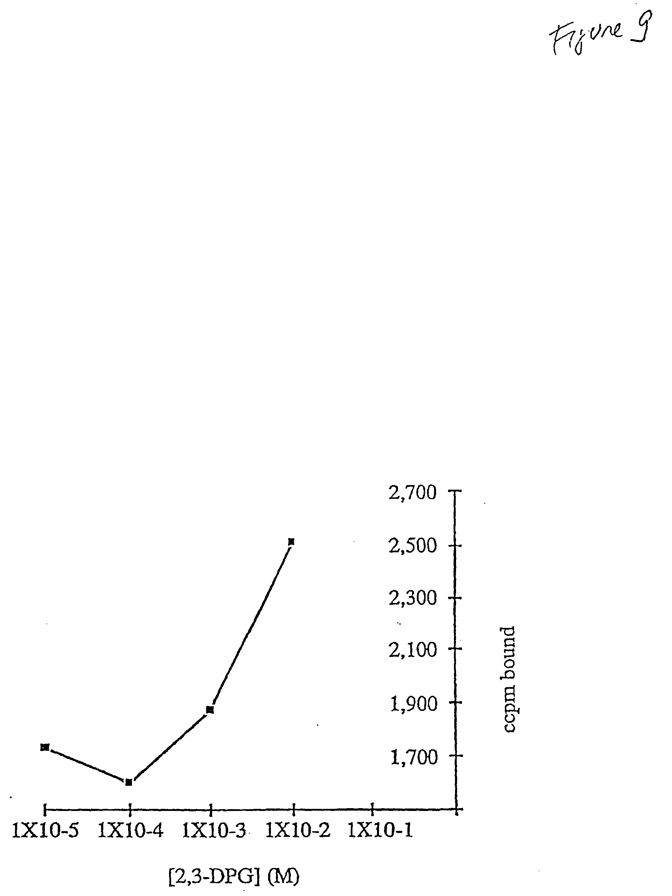

To validate the assay, a known ligand for hemoglobin, 2,3-diphosphoglycerate, was included in the reaction mixture at concentrations ranging from 10⁻⁵ to 10⁻¹M. As shown in Figure 8, 2,3-diphosphoglycerate significantly increased the filter retention of hemoglobin.

### B) ELISA Quantitation of Hemoglobin:

Reaction mixtures in a final volume of 0.05 ml contained 0.063 mg/ml Hemoglobin, 0.034 mg/ml Bovine Serum Albumin, 50 mM Tris-HCl, pH 7.5, 10 mM calcium acetate, 7.5 µg/ml thermolysin (Boeringer Mannheim), 7.5 µg/ml proteinase K (Merck), 10% DMSO, and the test compound at 20 or 200 µM concentration. Control reactions were identical, except that the test compound was omitted.

The mixtures were incubated at 20°C for 15 minutes, then at 44°C for 30 minutes, and then placed on ice. To each mixture was then added 0.05 ml 0.1M sodium borate buffer containing 20mM EDTA and 1mm PMSF. After 10 minutes incubation on ice, the mixtures were transferred to uncoated 96-well Immuulon-4 plates (Dynatech). The plates were then incubated at 4°C overnight to allow binding of the protein to the plate. The plates were washed thoroughly with TBST, and 0.1 ml of rabbit anti-human hemoglobin antibody (Calbiochem) dilute 1:500 was added to each well. The plates were incubated at room temperature for one hour, then thoroughly washed with TBST. Next 0.1 ml of alkaline phosphatase conjugated goat anti rabbit IgG antibody (Calbiochem) diluted 1:1000 in TBST plus 5% nonfat dry milk was added to each well and the plates were incubated at room temperature 1-2 hours. The plates were then washed thoroughly with TBST and finally with TBST lacking Tween. 0.1 ml per ml of *p*-nitrophenylphosphate (0.5 mg/ml) in 1X diethanolamine substrate buffer (Pierce) was added to each well. Plates were incubated at room temperature until color developed and the absorbance of each well at 405 nm was measured using a BioRad 3550-UV microplate reader.

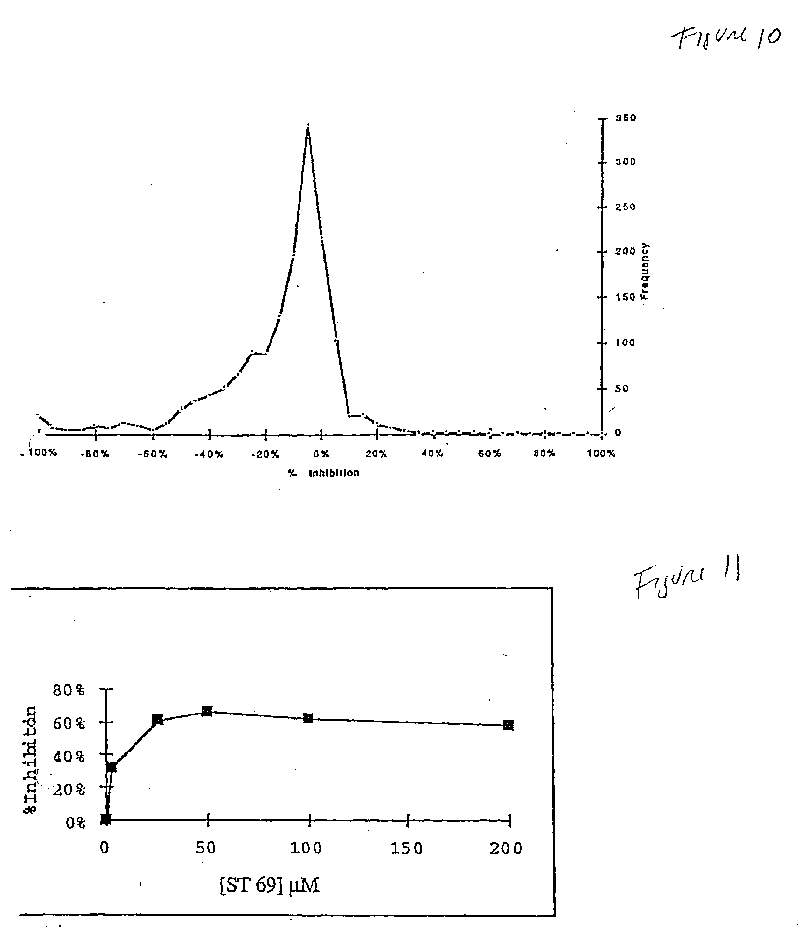

To validate the assay, a known ligand for hemoglobin, 2,3,-diphosphoglycerate, was included in the reaction. As shown in Figure 9, this compound increased the detection of immunoreactive hemoglobin.

### C) Results of High-throughput Screening:

4,000 compounds have been screened for interaction with HbS using proteolysis and ELISA detection as above (Figure 10). Of these, 23 were found to inhibit proteolysis to an extent of 20% or more when assayed at a concentration of 20 µM (positive hit compounds.)

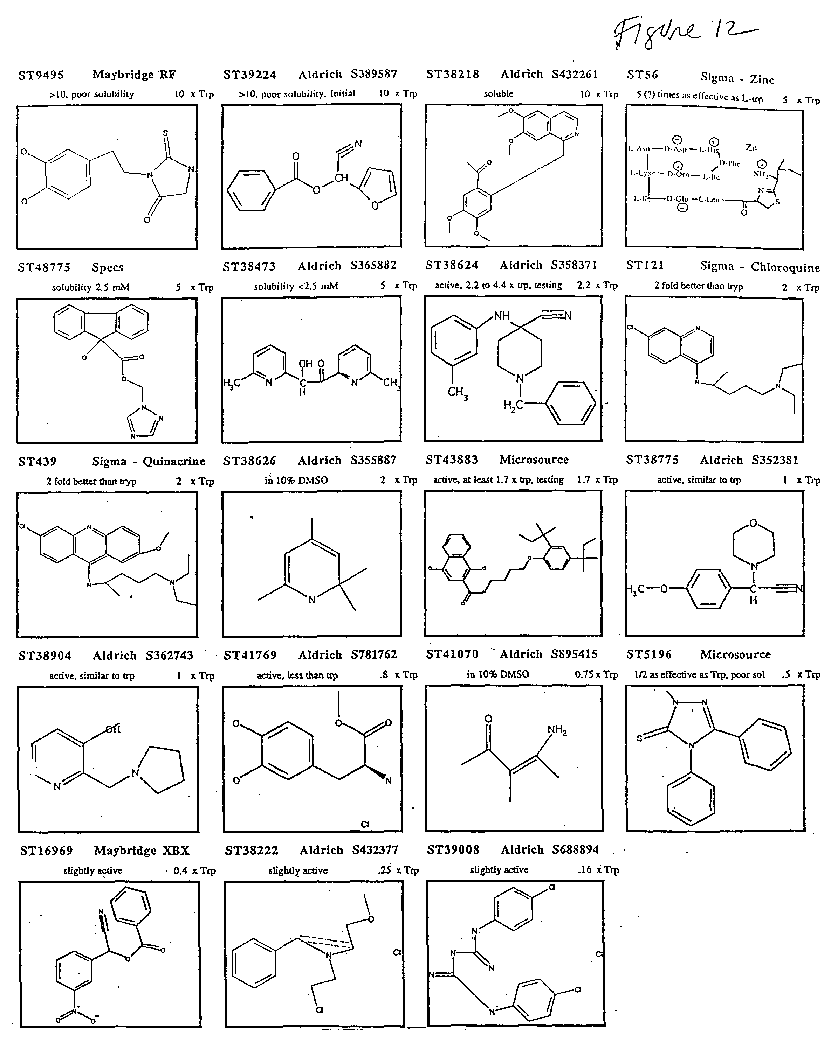

The concentration dependence of the effects of hit compounds was measured. Hit compounds showed half maximal effects at concentrations ranging as low as 2.0 µM (For example, see Figure 11).

### Example 12: High-Throughput Screening of Ligands for Human Met-Hemoglobin S

The experiments described below were performed to assay the binding of test ligands to Hemoblobin S. In these experiments, Hemoglobin S is included in its oxidized form, met-hemoglobin (met-HbS), wherein the heme iron is in the ferric state.

Reaction mixtures (in a final volume of 50 µl) contained 0.32 mg/ml hemoglobin S, 50 mM Tris-HCl, pH 7.5, 50 mM NaCl, 0.4 mM potassium ferricyanide, 40 µg/ml proteinase K, 10% DMSO, and the test compound at a concentration of 20 µM. Control reactions were identical, except that the test compound was omitted. Prior to the addition of proteinase K (0.2 µl) and DMSO (5 µl) or DMSO and test compound (5 µl) to the above reaction mixtures, the mixtures of hemoglobin S, Tris-HCl, NaCl, and potassium ferricyanide (in a volume of 44.8 µl) were incubated for 60 minutes on ice to allow the oxidation of the heme iron. The complete mixtures were then incubated at 20°C for 10 minutes, at 28.3°C for 30 minutes, and then placed on ice. Each mixture then received 150 µl of 50 mM sodium borate buffer, pH 8.5, containing 10 mM EDTA and 1.0 mM PMSF. After 10 minutes incubation on ice, 40 µl of the mixture were added to microplate wells containing 160 µl of BioRad™ protein assay reagent that had been diluted four-fold with water. After mixing, the absorbance at 585 nm was measured for each well.

Approximately 40,000 small molecules were screened in this manner at a rate of 3,000 to 7,000 assays per day. Of these, 108 compounds were found to inhibit proteolysis of met-HbS to an extent of between 15 and 100% when present at a concentration of 20 µM.

These "positive-hit" compounds were tested to determine whether they also inhibit proteolysis of a chromogenic peptide substrate, Succinyl-Ala-Ala-Ala-p-nitroanilide. Of these compounds, 51 reduced the rate chromogenic peptide hydrolysis at least two fold, indicating that their effect on HbS is non-specific, i.e., via inhibition of the protease rather than by binding to HbS.

The remaining 57 compounds were individually tested for their effects on the visible absorbance spectrum of met-HbS. This was done to identify those compounds whose apparent binding reflects contamination with cyanide or azide ions. Both cyanide and azide test positive using the assay of the present invention, which is presumably due to the fact that each binds with high affinity to the ferric iron atom of met-HbS and thereby increases met-HbS stability substantially. Binding of these ions to HbS also causes characteristic changes in its visible absorbance spectrum. The compounds were also tested for their ability to induce aggregation of met-HbS under the assay conditions used above; this was monitored by measuring changes in light scattering of the HbS solution at 595 nm.

Three compounds caused substantial aggregation and 23 others caused characteristic spectral changes. Thirty-one compounds caused neither aggregation nor spectral shifts that might indicate cyanide or azide contamination. These compounds represent ligands of HbS as defined by the methods of the present invention.

### Example 13: Activity of HbS Ligands in Inhibiting HbS Polymerization

Compounds identified as ligands of hemoglobin S (HbS) as described in Example 12 above were tested for their ability to influence the *in vitro* polymerization of HbS, using the C_{SAT} method. In this method, the effect of different agents on the equilibrium solubility (C_{SAT}) of HbS is assessed by measuring hemoglobin gelation.

### A. METHODS

1) *Purification of HbS:* HbS was purified from the blood of individuals homozygous for the sickle-cell trait. Blood was collected in heparin or EDTA tubes to prevent clotting. Erythrocytes were washed with cold phosphate-buffered saline that had been equilibrated with CO and were lysed with CO-equilibrated water. The lysate was desalted on a Sephadex G-25 column (Pharmacia) in CO-equilibrated 0.05M phosphate buffer, pH 7.0, and the hemoglobin concentration was adjusted to 15% (g/dl) tetramer.
   Further purification of HbS using pH gradient elution from DEAE-Sephadex was used for samples from heterozygotes or from individuals undergoing hydroxyurea treatment (which results in the production of HbF).
   The tetramer concentration was calculated using the following formula: C% (g/dl) = 64500 x 0.25 x 0.00001 x f x absorbance /∈ = 1.61 x 250 x absorbance / ∈, where
   f = dilution factor (usually 250),
   ∈ = extinction coefficients:
   ∈ (HbCO at 570 nm) = 14.2; ∈ (HbCO at 540 nm) = 14.3;
   ∈ (HbCO at 560 nm) = 12.1; ∈ (HbCO at 630 nm) = 0.20;
   ∈ (MetHb at 570 nm) = 3.63; ∈ (MetHb at 540 nm) = 5.96;
   ∈ (MetHb at 560 nm) = 3.72; ∈ (MetHb at 630 nm) = 3.88.

   Before use, 15% (g/dl) solutions of HbSCO are oxygenated for 2 hours, after which the extent of oxygenation was monitored spectroscopically (∈ (HbO₂ at 577 nm) = 14.6; ∈ (HbCO at 541 nm) = 13.8.) The oxygenated solutions are then brought to a concentration of 35% (g/dl) using Amicon cone or CENTRICON concentration (0°C, 3,500 rpm, 1.5 h).
2) *Gelation Assay:*
   Reaction mixtures contained 250 µl HbSO₂ (35% g/dl) and 80 µl of either buffer, control compounds (L-Phe or Trp), or test ligands. The mixtures were incubated on ice for 10 min, after which 10 µl of a cold solution of 0.9M sodium dithionite in nitrogen-purged phosphate buffer, pH 8.5, were added, and the mixtures incubated on ice for a further 10 minutes to convert oxy- to deoxy-HbS. The reaction mixtures were then incubated at 30°C to allow HbS gelation.

The reaction mixtures were then subjected to centrifugation at 35,000 rpm for 65 min at 30°C in a Beckmanh SW55Ti rotor. The supernatant of each mixture was then sampled in multiplicates and diluted 1:200 in Drabkins solution (Sigma Chemical Co., St. Louis, MO), and the absorbance of the diluted mixture at 540 nm was measured. The HbS concentration in the supernatant was calculated according to the following formula: HbS conc. = 1.61 x 200 x absorbance at 540 nm/ 11 = 29.32 x absorbance.

The solubility profiles were plotted using as the x axis the concentration of test ligand (mM) and as the y axis the C_{SAT} (the concentration of HbS in the supernatant in g/dl). (The C_{SAT} at 0 mM ligand should be about 16-18%.) The slope of each plot (dy/dx g/dl M) was then obtained, as well as the ratio of each slope to the control compounds (L-Phe and Trp). The slopes are directly proportional to molar effectiveness in inhibiting gelation.

### B. RESULTS:

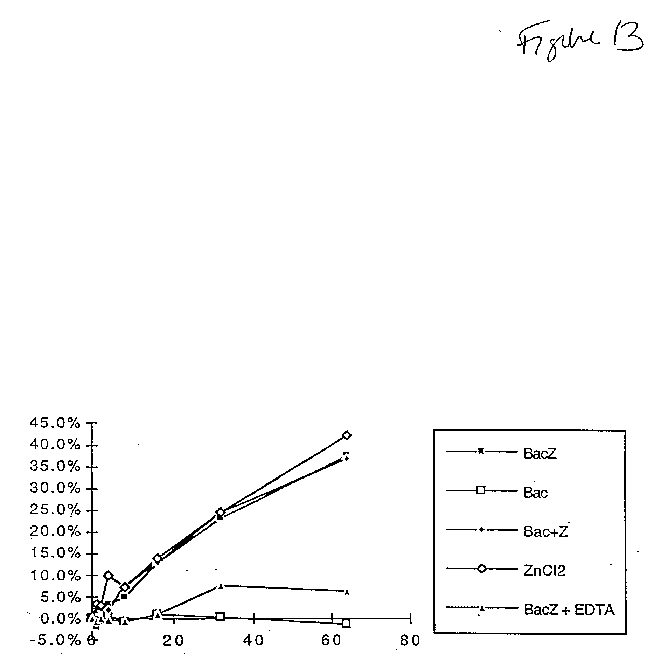

Of the 23 compounds identified using the methods of the present invention, 19 compounds showed detectable activity in inhibiting HbS gelation. Figure 12 shows the structures and commercial sources for these compounds and indicates their activities relative to Trp. Thirteen of these compounds exhibited anti-gelation activity at least equal to that of Trp.

For example, zinc-bacitracin (designated ST56 in Figure 12) is five times as effective as L-trp in inhibiting HbS polymerization. To investigate the ligand-binding properties of Zinc-bacitracin, Bacitracin and Zinc Chloride (ZnCl₂) were tested individually in the hemoglobin S binding assay described in Example 12 (Figure 13.) While zinc-free bacitracin is inactive, ZnCl₂ alone has an activity equivalent to that of zinc bacitracin. Thus Zn⁺⁺ is a ligand of hemoglobin and zinc-free bacitracin is not. Zinc-bacitracin complex itself may be a ligand of hemoglobin, or its activity may stem solely from liberation of Zn⁺⁺ from the complex.

Without wishing to be bound by theory, it is believed that the lack of activity in the remaining eight compounds may be due at least in part to their limited solubility at millimolar concentrations that are necessary in the gelation assay.

In summary, the methods of the present invention were successful in identifying a discrete number of HbS ligands from among 40,000 test ligands.

## Claims

1. A method for rapid, large-scale screening to identify a ligand that binds to a predetermined target protein, comprising the steps of:
(a) selecting as test ligands a plurality of compounds not known to bind to the target protein;
(b) incubating each of said test ligands and the target protein to produce a test combination;
(c) incubating the target protein in the absence of a test ligand to produce a control combination;
(d) treating the test and control combinations to cause the target protein in the control combination to unfold to a measurable extent;
(e) determining the extent to which the target protein occurs in the folded state, the unfolded state or both in the test combination and in the control combination;
(f) comparing the determination made in step (e) between the test and control combinations, wherein if the target protein is present in the folded state to a greater extent in the test combination than in the control combination, the test ligand is a ligand that binds to the target protein; and
(g) repeating steps (b)-(f) with a plurality of said test ligands until a ligand that binds to the target protein is identified;
with the proviso that the treatment step (d) does not comprise a contact of said test and control combinations with a conformational-sensitive fluorescence probe.

2. The method of claim 1, wherein the treating step comprises at least one of altering the temperature, adding denaturing compounds, and combinations thereof.

3. The method of claim 2, wherein the treating step comprises adding denaturing compounds and the denaturing compounds are selected from the group consisting of urea, guanidinium and salts thereof, detergents, and combinations thereof.

4. A method according to claim 1 or 2, wherein the treating step includes subjecting the test and control combinations to increased temperature, thereby causing a detectable fraction of said target to exist in an unfolded state.

5. A method for rapid, large-scale screening to identify a ligand that binds to a predetermined target protein, comprising the steps of:
(a) selecting as test ligands a plurality of compounds not known to bind to the target protein;
(b) incubating each of said test ligands and the target protein under conditions appropriate for the target protein to unfold to a measurable extent, thereby producing a test combination;
(c) incubating the target protein as in step (b), but in the absence of a test ligand, to produce a control combination;
(d) determining the extent to which the target protein occurs in the folded state, the unfolded state or both in the test combination and in the control combination;
(e) comparing the determination made in step (d) between the test and control combinations, wherein if the target protein is present in the folded state to a greater extent in the test combination than in the control combination, the test ligand is a ligand that binds to the target protein; and
(f) repeating steps (b)-(e) with a plurality of said test ligands until a ligand that binds to the target protein is identified;
with the proviso that the incubation steps (b) and (c) do not comprise a contact of said test and control combinations with a conformational-sensitive fluorescence probe.

6. The method of claim 5, wherein the ligand binds to surface or internal amino acid residues or conformational domains of the target protein.

7. The method of any one of claims 1-6, wherein the determining step comprises subjecting the test and control combinations to at least one of the proteolysis; antibody binding; surface binding; molecular chaperone binding; binding to a known ligand, cofactor, substrate, or analogue thereof of the target protein; circular dichroism spectroscopy; ultraviolet spectroscopy; fluorescence spectroscopy; calorimetry; and combinations thereof.

8. The method of Claim 7, wherein the determining step comprises the steps of:
(i) incubating the target protein, each of said test ligands and one or more proteases which preferentially degrade target protein in its unfolded state, whereby the target protein in its unfolded state is degraded preferentially; and
(ii) measuring the fraction of the target protein degraded or the fraction of the target protein remaining in an intact state, wherein if the fraction of target protein remaining in the intact state in the test combination is greater than in the control combination, then the test ligand is a ligand that binds to the target protein.

9. The method of Claim 7, wherein the determining step comprises the steps of:
(i) combining the target protein and each of said test ligands;
(ii) exposing the target protein and the test ligand mixture to a surface which preferentially binds unfolded target protein, whereby unfolded target protein binds to the surface; and
(iii) determining the fraction of the target protein bound to the surface or the fraction of the target protein remaining unbound, wherein if the fraction of target protein remaining unbound is greater in the test combination, than in the control combination, the test ligand is a ligand that binds to the target protein.

10. The method of Claim 7, wherein the determining step comprises the steps of:
(i) combining the target protein and each of said test ligands on a surface to which a known ligand of the target protein has been immobilized; and
(ii) determining the fraction of the target protein bound to the surface or the fraction of the target protein remaining unbound wherein if the fraction of target protein bound to the surface is greater in the test combination than in the control combination, the test ligand is a ligand that binds to the target protein.

11. The method of Claim 7, wherein the determining step comprises using antibodies that distinguish between the folded form and the unfolded form of the target protein.

12. The method of Claim 11, wherein the determining step comprises the steps of:
(i) coating a surface with a specific antibody directed against the denatured state of the target protein;
(ii) incubating the target protein in the presence of each of said test ligands on the surface; and
(iii) determining the fraction of the target protein bound to or not bound to the surface.

13. The method of Claim 11, wherein the determining step comprises the steps of:
(i) coating a surface with an antibody capable of binding the target protein in its native state or denatured state;
(ii) incubating the target protein in the presence of each of said test ligands and an antibody capable of binding the target protein in its folded state or in the other of its native and denatured states; and
(iii) determining the presence of the target protein bound or remaining unbound to the surface.

14. The method of Claim 7, wherein the determining step comprises determining the amount of target protein bound to a molecular chaperone protein or not bound to a molecular chaperone protein.

15. The method of Claim 14, wherein the determining step comprises the steps of:
(i) coating a surface with a molecular chaperone protein;
(ii) incubating the target protein and each of said test ligands on the coated surface; and
(iii) determining the amount of the target protein bound to or remaining unbound to the surface.

16. The method of Claim 14, wherein the determining step comprises the steps of:
(i) coating a surface with the target protein in a denatured state;
(ii) incubating a purified form of the target protein in the presence of each of said test ligands and a molecular chaperone protein on the surface; and
(iii) determining the amount of either or both of the molecular chaperone protein and the target protein remaining unbound to or binding to the surface.

17. The method of Claim 7, wherein the determining step comprises determining differential formation of aggregated protein using a method selected from the group consisting of:
(i) measuring the amount of aggregated protein;
(ii) measuring the amount of soluble protein and measuring the rate of formation of aggregated protein.

18. The method of Claim 7, wherein said binding to a known ligand, cofactor, substrate, or analogues thereof comprises the steps of:
(i) immobilizing the known ligand, cofactor, substrate, or analgoue thereof on a solid support;
(ii) exposing the solid support to the test and control combinations, under conditions in which the target protein in its folded state binds to the support; and
(iii) determining the fraction of the target protein bound to the support in the test and control combinations,
the ligand being any test ligand that causes an increase or decrease in said fraction in the test combination relative to said fraction in the control combination.

19. The method of claim 8, wherein the proteases of the incubating step are selected from the group consisting of proteinase K, thermolysin, and combinations thereof.

20. The method of claim 14, wherein the measuring step comprises subjecting the test and control combinations to denaturing polyacrylamide gel electrophoresis.

21. The method of Claim 7, wherein the determining step comprises the steps of:
(i) coating a surface with antisera capable of binding to the folded target protein;
(ii) incubating the target protein in the presence of a molecular chaperone and each of said test ligands on the surface; and
(iii) determining the amount of the target protein binding to or remaining unbound to the surface.

22. A method according to any of claims 1-21 wherein the target protein in the control combination unfolds irreversibly.

23. A method according to any of claims 1-21 wherein the target protein in the control combination unfolds reversibly.

24. A method according to any of claims 1-23 wherein the target protein is selected from the group consisting of carbonic anhydrase, human neutrophil elastase, human hemoglobin, dihydrofolate reductase, and HIV Rev protein.

## Patentansprüche

1. Verfahren zum schnellen, großangelegten Absuchen zur Identifizierung eines Liganden, der an ein vorher bestimmtes Zielprotein bindet, wobei das Verfahren die folgenden Schritte umfasst:
(a) Auswahl einer großen Anzahl von Verbindungen als Testliganden, von denen nicht bekannt ist, ob sie an das Zielprotein binden;
(b) Inkubation jedes der Testliganden und des Zielproteins, wodurch eine Testzusammensetzung hergestellt wird;
(c) Inkubation des Zielproteins in Abwesenheit eines Testliganden, wodurch eine Kontrollzusammensetzung hergestellt wird;
(d) Behandlung der Test- und Kontrollzusammensetzungen, wodurch bewirkt wird, dass sich das Zielprotein in der Kontrollzusammensetzung bis zu einem messbaren Ausmaß entfaltet;
(e) Bestimmung des Ausmaßes, mit dem das Zielprotein im gefalteten Zustand, im ungefalteten Zustand oder in beiden in der Testzusammensetzung und in der Kontrollzusammensetzung vorliegt;
(f) Vergleich der in Schritt (e) durchgeführten Bestimmung der Test- und Kontrollzusammensetzungen, wobei der Testligand ein Ligand ist, der an das Zielprotein bindet, wenn das Zielprotein in der Testzusammensetzung in einem größeren Ausmaß im gefalteten Zustand vorliegt als in der Kontrollzusammensetzung; und
(g) Wiederholung der Schritte (b) bis (f) mit einer großen Anzahl von Testliganden bis ein Ligand identifiziert ist, der an das Zielprotein bindet;
mit der Maßgabe, dass der Behandlungsschritt (d) keinen Kontakt der Test- und Kontrollzusammensetzungen mit einer für die Konformation empfindlichen Fluoreszenzsonde umfasst.

2. Verfahren nach Anspruch 1, wobei der Behandlungsschritt mindestens einen der folgenden Schritte umfasst: Änderung der Temperatur, Zugabe von Denaturierungsmitteln und Kombinationen davon.

3. Verfahren nach Anspruch 2, wobei der Behandlungsschritt die Zugabe von Denaturierungsmitteln umfasst und die Denaturierungsmittel aus einer Gruppe ausgewählt sind, die aus Harnstoff, Guanidinium und Salzen davon, Detergenzien und Kombinationen davon besteht.

4. Verfahren nach Anspruch 1 oder 2, wobei der Behandlungsschritt umfasst, dass die Test- und Kontrollzusammensetzungen einer erhöhten Temperatur unterworfen werden, wodurch bewirkt wird, dass eine nachweisbare Fraktion der Zielsubstanz in einem ungefalteten Zustand vorliegt.

5. Verfahren zum schnellen, großangelegten Absuchen zur Identifizierung eines Liganden, der an ein vorher bestimmtes Zielprotein bindet, wobei das Verfahren die folgenden Schritte umfasst:
(a) Auswahl einer großen Anzahl von Verbindungen als Testliganden, von denen nicht bekannt ist, ob sie an das Zielprotein binden;
(b) Inkubation jedes der Testliganden und des Zielproteins unter Bedingungen, die dafür geeignet sind, dass das Zielprotein bis zu einem messbaren Ausmaß entfaltet wird, wodurch eine Testzusammensetzung hergestellt wird;
(c) Inkubation des Zielproteins wie in Schritt (b), jedoch in Abwesenheit eines Testliganden, wodurch eine Kontrollzusammensetzung hergestellt wird;
(d) Bestimmung des Ausmaßes, bis zu dem das Zielprotein im gefalteten Zustand, im entfalteten Zustand oder in beiden in der Testzusammensetzung und in der Kontrollzusammensetzung vorliegt;
(e) Vergleich der in Schritt (d) durchgeführten Bestimmung der Test- und Kontrollzusammensetzungen, wobei der Testligand ein Ligand ist, der an das Zielprotein bindet, wenn das Zielprotein in der Testzusammensetzung in einem größeren Ausmaß im gefalteten Zustand vorliegt als in der Kontrollzusammensetzung; und
(f) Wiederholung der Schritte (b) bis (e) mit einer großen Anzahl von Testliganden bis ein Ligand identifiziert ist, der an das Zielprotein bindet;
mit der Maßgabe, dass die Inkubationsschritte (b) und (c) keinen Kontakt der Test- und Kontrollzusammensetzungen mit einer für die Konformation empfindlichen Fluoreszenzsonde umfassen.

6. Verfahren nach Anspruch 5, wobei der Ligand an Oberflächen- oder innere Aminosäurereste oder die Konformation betreffende Domänen des Zielproteins bindet.

7. Verfahren nach einem der Ansprüche 1 bis 6, wobei der Bestimmungsschritt umfasst, dass die Test- oder Kontrollzusammensetzungen mindestens einem der folgenden Verfahren unterworfen werden: Proteolyse; Antikörperbindung; Oberflächenbindung; molekulare Chaperon-Bindung; Bindung an einen bekannten Liganden, Cofaktor, Substrat oder Analogon davon des Zielproteins; Circulardichroismus-Spektroskopie; Ultraviolett-Spektroskopie; Fluoreszenz-Spektroskopie; Kalorimetrie und Kombinationen davon.

8. Verfahren nach Anspruch 7, wobei der Bestimmungsschritt die folgenden Schritte umfasst:
(i) Inkubation des Zielproteins, jedes der Testliganden und einer oder mehrerer Proteasen, die vorzugsweise das Zielprotein im ungefalteten Zustand spalten, wodurch das Zielprotein im ungefalteten Zustand vorzugsweise gespalten wird; und
(ii) Messung der Fraktion des gespaltenen Zielproteins oder der Fraktion des in einem intakten Zustand verbleibenden Zielproteins, wobei der Testligand ein Ligand ist, der an das Zielprotein bindet, wenn die Fraktion des im intakten Zustand verbleibenden Zielproteins in der Testzusammensetzung größer ist als in der Kontrollzusammensetzung.

9. Verfahren nach Anspruch 7, wobei der Bestimmungsschritt die folgenden Schritte umfasst:
(i) Vereinigung des Zielproteins und jedes der Testliganden;
(ii) Inkontaktbringen des Gemisches aus Zielprotein und Testligand mit einer Oberfläche, die vorzugsweise ungefaltetes Zielprotein bindet, wodurch ungefaltetes Zielprotein an die Oberfläche bindet; und
(iii) Bestimmung der Fraktion des an die Oberfläche gebundenen Zielproteins oder der Fraktion des ungebunden verbleibenden Zielproteins, wobei der Testligand ein Ligand ist, der an das Zielprotein bindet, wenn die Fraktion des ungebunden verbleibenden Zielproteins in der Testzusammensetzung größer ist als in der Kontrollzusammensetzung.

10. Verfahren nach Anspruch 7, wobei der Bestimmungsschritt die folgenden Schritte umfasst:
(i) Vereinigung des Zielproteins und jedes der Testliganden auf einer Oberfläche, an die ein bekannter Ligand des Zielproteins immobilisiert wurde; und
(ii) Bestimmung der Fraktion des an die Oberfläche gebundenen Zielproteins oder der Fraktion des ungebunden verbleibenden Zielproteins, wobei der Testligand ein Ligand ist, der an das Zielprotein bindet, wenn die Fraktion des an die Oberfläche gebundenen Zielproteins in der Testzusammensetzung größer ist als in der Kontrollzusammensetzung.

11. Verfahren nach Anspruch 7, wobei der Bestimmungsschritt die Verwendung von Antikörpern umfasst, die zwischen der gefalteten Form und der ungefalteten Form des Zielproteins unterscheiden.

12. Verfahren nach Anspruch 11, wobei der Bestimmungsschritt die folgenden Schritte umfasst:
(i) Beschichtung einer Oberfläche mit einem spezifischen Antikörper, der gegen die denaturierte Form des Zielproteins gerichtet ist;
(ii) Inkubation des Zielproteins in Gegenwart jedes der Testliganden auf der Oberfläche; und
(iii) Bestimmung der Fraktion des an die Oberfläche gebundenen oder nicht daran gebundenen Zielproteins.

13. Verfahren nach Anspruch 11, wobei der Bestimmungsschritt die folgenden Schritte umfasst:
(i) Beschichtung einer Oberfläche mit einem Antikörper, der zur Bindung des Zielproteins im nativen Zustand oder im denaturierten Zustand fähig ist;
(ii) Inkubation des Zielproteins in Gegenwart jedes der Testliganden und eines Antikörpers, der zur Bindung des Zielproteins im gefalteten Zustand oder in einem anderen nativen oder denaturierten Zustand fähig ist; und
(iii) Bestimmung des Vorliegens des an die Oberfläche gebundenen oder ungebunden darauf verbleibenden Zielproteins.

14. Verfahren nach Anspruch 7, wobei der Bestimmungsschritt die Bestimmung der Menge des Zielproteins umfasst, das an ein molekulares Chaperon-Protein gebunden ist oder das nicht an ein molekulares Chaperon-Protein gebunden ist.

15. Verfahren nach Anspruch 14, wobei der Bestimmungsschritt die folgenden Schritte umfasst:
(i) Beschichtung einer Oberfläche mit einem molekularen Chaperon-Protein;
(ii) Inkubation des Zielproteins und jedes der Testliganden auf der beschichteten Oberfläche; und
(iii) Bestimmung der Menge des an die Oberfläche gebundenen oder ungebunden darauf verbleibenden Zielproteins.

16. Verfahren nach Anspruch 14, wobei der Bestimmungsschritt die folgenden Schritte umfasst:
(i) Beschichtung einer Oberfläche mit dem Zielprotein in einem denaturierten Zustand;
(ii) Inkubation einer gereinigten Form des Zielproteins in Gegenwart jedes der Testliganden und eines molekularen Chaperon-Proteins auf der Oberfläche; und
(iii) Bestimmung der Menge von einem oder von beiden des molekularen Chaperon-Proteins und des ungebunden auf der Oberfläche verbleibenden oder daran bindenden Zielproteins.

17. Verfahren nach Anspruch 7, wobei der Bestimmungsschritt die Bestimmung der charakteristischen Bildung von aggregiertem Protein unter Verwendung eines Verfahrens umfasst, das aus der folgenden Gruppe ausgewählt ist:
(i) Messung der Menge von aggregiertem Protein;
(ii) Messung der Menge von löslichem Protein und Messung der Rate der Bildung von aggregiertem Protein.

18. Verfahren nach Anspruch 7, wobei die Bindung an einen bekannten Liganden, Cofaktor, Substrat oder Analoga davon die folgenden Schritte umfasst:
(i) Immobilisierung des bekannten Liganden, Cofaktors, Substrats oder Analogons davon an einen festen Träger;
(ii) Inkontaktbringen des festen Trägers mit den Test- und Kontrollzusammensetzungen unter Bedingungen, bei denen das Zielprotein im gefalteten Zustand an den Träger bindet; und
(iii) Bestimmung der Fraktion des an den Träger gebundenen Zielproteins in den Test- und Kontrollzusammensetzungen,
wobei der Ligand ein beliebiger Testligand ist, der eine Zunahme oder Abnahme dieser Fraktion in der Testzusammensetzung im Vergleich zu dieser Fraktion in der Kontrollzusammensetzung bewirkt

19. Verfahren nach Anspruch 8, wobei die Proteasen des Inkubationsschrittes aus der Gruppe ausgewählt sind, die aus Proteinase K, Thermolysin und Kombinationen davon besteht

20. Verfahren nach Anspruch 14, wobei der Messungsschritt umfasst, dass die Test- und Kontrollzusammensetzungen einer denaturierenden Polyacrylamidgel-Elektrophorese unterworfen werden.

21. Verfahren nach Anspruch 7, wobei der Bestimmungsschritt die folgenden Schritte umfasst:
(i) Beschichtung einer Oberfläche mit Antiseren, die zur Bindung an das gefaltete Zielprotein fähig sind;
(ii) Inkubation des Zielproteins in Gegenwart eines molekularen Chaperons und jedes der Testliganden auf der Oberfläche; und
(iii) Bestimmung der Menge des an die Oberfläche bindenden oder ungebunden darauf verbleibenden Zielproteins.

22. Verfahren nach einem der Ansprüche 1 bis 21, wobei sich das Zielprotein in der Kontrollzusammensetzung irreversibel entfaltet

23. Verfahren nach einem der Ansprüche 1 bis 21, wobei sich das Zielprotein in der Kontrollzusammensetzung reversibel entfaltet.

24. Verfahren nach einem der Ansprüche 1 bis 23, wobei das Zielprotein aus einer Gruppe ausgewählt ist, die aus Kohlensäureanhydrase, menschlicher neutrophiler Elastase, menschlichem Hämoglobin, Dihydrofolatreduktase und HIV-Rev-Protein besteht.

## Revendications

1. Méthode de sélection rapide, à grande échelle, pour identifier un ligand qui se lie à une protéine cible prédéterminée, comprenant les étapes consistant :
(a) à sélectionner, en tant que ligands à tester, une pluralité de composés non connus pour se lier à la protéine cible ;
(b) à incuber chacun desdits ligands à tester et la protéine cible pour produire une combinaison de test ;
(c) à incuber la protéine cible en l'absence d'un ligand à tester pour produire une combinaison témoin ;
(d) à traiter les combinaisons de test et témoin pour amener la protéine cible dans la combinaison témoin à se déplier dans une proportion mesurable ;
(e) à déterminer dans quelle mesure la protéine cible est présente à l'état plié, à l'état déplié ou les deux dans la combinaison de test et dans la combinaison témoin ;
(f) à comparer la détermination faite dans l'étape (e) dans les combinaisons test et témoin, le ligand testé étant, dans le cas où la protéine cible est présente à l'état plié dans une plus grande proportion dans la combinaison de test que dans la combinaison témoin, un ligand qui se lie à la protéine cible ; et
(g) à répéter les étapes (b)-(f) avec plusieurs desdits ligands testés jusqu'à ce que l'on identifie un ligand qui se lie à la protéine cible, sous réserve que l'étape de traitement (d) ne comprenne pas de contact desdites combinaisons test et témoin avec une sonde fluorescente sensible à la conformation.

2. Méthode selon la revendication 1, dans laquelle l'étape de traitement comprend au moins une modification de la température, une addition de composés dénaturants et des combinaisons de ces opérations.

3. Méthode selon la revendication 2, dans laquelle l'étape de traitement comprend l'addition de composés dénaturants, ces derniers étant choisis dans le groupe constitué par l'urée, le guanidium et ses sels, des détergents, et des combinaisons de ces composants.

4. Méthode selon la revendication 1 ou 2, dans laquelle l'étape de traitement comprend le fait de soumettre les combinaisons de test et témoin à une augmentation de la température, amenant ainsi une fraction détectable de ladite cible à se trouver sous forme dépliée.

5. Méthode de sélection rapide, à grande échelle, pour identifier un ligand qui se lie à une protéine cible prédéterminée, comprenant les étapes consistant :
(a) à sélectionner, en tant que ligands à tester, une pluralité de composés non connus pour se lier à la protéine cible ;
(b) à incuber chacun desdits ligands à tester et la protéine cible dans des conditions appropriées pour que la protéine cible puisse se déplier dans une proportion mesurable, produisant ainsi une combinaison de test ;
(c) à incuber la protéine cible comme dans l'étape (b), mais en l'absence d'un ligand à tester, pour produire une combinaison témoin ;
(d) à déterminer dans quelle mesure la protéine cible est présente à l'état plié, à l'état déplié ou les deux dans la combinaison de test et dans la combinaison témoin ;
(e) à comparer les déterminations faites dans l'étape (d) dans les combinaisons test et témoin, le ligand testé étant, dans le cas où la protéine cible est présente à l'état plié dans une plus grande proportion dans la combinaison de test que dans la combinaison témoin, un ligand qui se lie à la protéine cible ; et
(f) à répéter les étapes (b)-(e) avec plusieurs desdits ligands testés jusqu'à ce que l'on identifie un ligand qui se lieaà la protéine cible ; sous réserve que les étapes d'incubations (b) et (c) ce comprennent pas de contact desdites combinaisons test et témoin avec une sonde fluorescente sensible à la conformation.

6. Méthode selon la revendication 5, dans laquelle le ligand se lie à la surface, à des résidus d'acides aminés internes ou à des domaines conformationnels de la protéine cible.

7. Méthode selon l'une quelconque des revendications 1-6, dans laquelle l'étape de détermination comprend le fait de soumettre les combinaisons de test et témoin à au moins une protéolyse ; une liaison d'anticorps ; une liaison à une surface ; une liaison moléculaire à une chaperone ; une liaison à un ligand, à un cofacteur ou à un substrat connu, ou à un de leurs analogues, de la protéine cible; une spectroscopie à dichroïsme circulaire; une spectroscopie dans l'ultraviolet ; une spectroscopie en fluorescence ; une calorimétrie ; et leurs combinaisons.

8. Méthode selon la revendication 7, dans laquelle l'étape de détermination comprend les étapes consistant :
(i) à incuber la protéine cible, chacun desdits ligands à tester et une ou plusieurs protéases qui dégradent de préférence la protéine cible à l'état déplié, la protéine cible étant ainsi dégradée de façon préférentielle dans son état déplié ; et
(ii) à mesurer la fraction de la protéine cible dégradée ou la fraction de la protéine cible restant à l'état intact, le ligand testé étant, dans le cas où la fraction de la protéine cible restant à l'état intact dans la combinaison de test est plus grande que celle dans la combinaison témoin, un ligand qui se lie à la protéine cible.

9. Méthode selon la revendication 7, dans laquelle l'étape de détermination comprend les étapes consistant :
(i) à combiner la protéine cible et chacun desdits ligands à tester ;
(ii) à exposer le mélange de la protéine cible et du ligand à tester à une surface qui se lie préférentiellement à une protéine cible dépliée, la protéine cible dépliée se liant préférentiellement à la surface ; et
(iii) à déterminer la fraction de la protéine liée à la surface ou la fraction de la protéine cible restant à l'état non lié, le ligand testé étant, dans le cas où la fraction de la protéine cible restant à l'état non lié dans la combinaison de test est plus grande que celle dans la combinaison témoin, un ligand qui se lie à la protéine cible.

10. Méthode selon la revendication 7, dans laquelle l'étape de détermination comprend les étapes consistant :
(i) à combiner la protéine cible et chacun desdits ligands à tester à une surface sur laquelle un ligand connu de la protéine cible a été immobilisé ; et
(ii) à déterminer la fraction de la protéine cible liée à la surface ou la fraction de la protéine cible restant à l'état non lié, le ligand testé étant, dans le cas où la fraction de la protéine cible lié à la surface dans la combinaison de test est plus grande que celle dans la combinaison témoin, un ligand qui se lie à la protéine cible.

11. Méthode selon la revendication 7, dans laquelle l'étape de détermination comprend l'utilisation d'anticorps qui distinguent entre la forme pliée et la forme dépliée de la protéine cible.

12. Méthode selon la revendication 11, dans laquelle l'étape de détermination comprend les étapes consistant :
(i) à revêtir une surface avec un anticorps spécifique dirigé contre l'état dénaturé de la protéine cible ;
(ii) à incuber la protéine cible en présence de chacun desdits ligands à la surface ; et
(iii) à déterminer la fraction de la protéine cible liée ou non liée à la surface.

13. Méthode selon la revendication 11, dans laquelle l'étape de détermination comprend les étapes consistant :
(i) à revêtir la surface avec un anticorps capable de se lier à la protéine cible à l'état naturel ou à l'état dénaturé ;
(ii) à incuber la protéine cible en présence de chacun desdits ligands à tester et d'un anticorps capable de se lier à la protéine cible à l'état plié ou à l'autre de ses états natif et dénaturé ; et
(iii) à déterminer la présence de la protéine cible liée à la surface ou restant non liée.

14. Méthode selon la revendication 7, dans laquelle l'étape de détermination comprend la détermination de la quantité de protéine cible liée à une protéine chaperone moléculaire ou non liée à une protéine chaperone moléculaire.

15. Méthode selon la revendication 14, dans laquelle l'étape de détermination comprend les étapes consistant :
(i) à revêtir une surface avec une protéine chaperone moléculaire ;
(ii) à incuber la protéine cible et chacun desdits ligands à tester sur la surface revêtue ; et
(iii) à déterminer la quantité de la protéine cible liée à la surface ou restant non liée.

16. Méthode selon la revendication 14, dans laquelle l'étape de détermination comprend les étapes consistant :
(i) à revêtir une surface avec la protéine cible à l'état dénaturé ;
(ii) à incuber une forme purifiée de la protéine cible en présence de chacun desdits ligands à tester et une protéine chaperone moléculaire sur la surface ; et
(iii) à déterminer la quantité de l'une ou des deux, de la protéine chaperone moléculaire et de la protéine cible, restant non liée ou liée à la surface.

17. Méthode selon la revendication 7, dans laquelle l'étape de détermination comprend la détermination de la formation différentielle de protéine agrégée, en utilisant une méthode choisie dans le groupe constitué par :
(i) la mesure de la quantité de protéine agrégée ;
(ii) la mesure de la quantité de protéine soluble et la mesure de la vitesse de formation de protéine agrégée.

18. Méthode selon la revendication 7, dans laquelle ladite liaison à un ligand, un cofacteur ou un substrat connu, ou à un analogue de ceux-ci, comprend les étapes consistant :
(i) à immobiliser le ligand, cofacteur ou substrat connu, ou l'analogue de ceux-ci, sur un support solide ;
(ii) à exposer le support solide aux combinaisons de test et témoin, dans des conditions dans lesquelles la protéine cible à l'état plié se lie au support ; et
(iii) à déterminer la fraction de la protéine cible liée au support dans les combinaisons de test et témoin,
le ligand étant l'un quelconque des ligands testés qui provoque une augmentation ou une diminution de ladite fraction dans la combinaison de test par rapport à ladite fraction dans la combinaison témoin.

19. Méthode selon la revendication 8, dans laquelle les protéases de l'étape d'incubation sont choisies dans le groupe constitué par la protéinase K, la thermolysine et leurs combinaisons.

20. Méthode selon la revendication 14, dans laquelle l'étape de détermination comprend le fait de soumettre les combinaisons de test et témoin à une dénaturation par électrophorèse sur gel de polyacrylamide.

21. Méthode selon la revendication 7, dans laquelle l'étape de détermination comprend les étapes consistant :
(i) à revêtir une surface avec des antisérums capables de se lier à la protéine cible à l'état plié ;
(ii) à incuber la protéine cible en présence d'une chaperone moléculaire et de chacun desdits ligands à tester et sur la surface ; et
(iii) à déterminer la quantité de la protéine cible liée à la surface ou restant non liée.

22. Méthode selon l'une quelconque des revendications 1-21, dans laquelle la protéine cible dans la combinaison témoin se déplie de manière irréversible.

23. Méthode selon l'une quelconque des revendications 1-21, dans laquelle la protéine cible dans la combinaison témoin se déplie de manière réversible.

24. Méthode selon l'une quelconque des revendications 1-23, dans laquelle la protéine cible est choisie dans le groupe constitué par l'anhydrase carbonique, l'élastase neutrophile humaine, l'hémoglobine humaine, la dihydrofolate réductase et la protéine HIV Rev.
